# EUROPÄISCHE PATENTSCHRIFT

(11) **EP 3 911 387 B1**
(45) Veröffentlichungstag und Bekanntmachung des Hinweises auf die Patenterteilung: **19.04.2023**
(21) Anmeldenummer: 20700882.2
(22) Anmeldetag: 14.01.2020
(51) Int. Cl.: A61M 15/00, B05B 11/06

(54) **SUBSTANZBEHÄLTER FÜR EINE VORRICHTUNG ZUM INHALIEREN PULVERFÖRMIGER SUBSTANZEN**
SUBSTANCE CONTAINER FOR A DEVICE FOR INHALING POWDER-TYPE SUBSTANCES
RÉSERVOIR DE SUBSTANCE POUR UN DISPOSITIF POUR INHALER DES SUBSTANCES PULVÉRULENTES

(30) Priorität: 14.01.2019 DE 102019100832; 13.01.2020 DE 102020100550
(43) Veröffentlichungstag der Anmeldung: 24.11.2021
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2020/050808
(87) Internationale Veröffentlichungsnummer: WO 2020/148276

(56) Entgegenhaltungen:
- EP-A2- 0 211 595
- WO-A1-2004/045688
- WO-A2-02/053216
- US-A1- 2007 131 225
- US-A1- 2010 294 278
- US-B2- 7 571 724

## Beschreibung

### Gebiet der Technik

Die Erfindung betrifft einen Substanzbehälter für eine Vorrichtung zum Inhalieren pulverförmiger Substanzen nach den Merkmalen des Oberbegriffes des Anspruches 1.

### Stand der Technik

Bekannt sind Substanzbehälter, die in einer Vorrichtung hintereinander angeordnet vorgesehen sind. Dabei können die Substanzbehälter untereinander unverbunden sein, beispielsweise zufolge Ausgestaltung der Substanzbehälter in Art einer Blisterpackung oder eines Blisterstreifens.

Die Substanzbehälter werden mit jeder Betätigung der Vorrichtung sukzessive in die Entleerungsstellung verlagert.

Eine Vorrichtung, in welcher eine Vielzahl von Substanzbehältern aufgenommen ist, die zur Entleerung sukzessive in eine Entleerungsposition verbracht werden, ist beispielsweise aus der WO 2005/049121 A1 (US 2007/0131225 A1) bekannt. Die Substanzbehälter werden durch Antriebsräder in einer Endlosschlange fortbewegt. Ein Substanzbehälter befindet sich in einer nicht näher beschriebenen Entleerungsstellung außerhalb jedes der Antriebsräder.

Aus der WO 2003/061 743 A1 (US 8 511 304 B2) ist es bekannt, dass die Substanzbehälter in einer Vorrichtung kettenartig in Art eines Streifen-Blisters miteinander verbunden, vorgesehen sind, wobei die Substanzbehälter so ausgebildet sind, dass diese zwei gegebenenfalls, wie auch bevorzugt, unterschiedliche Substanzen in vorgegebenen Dosierungen bevorraten. Diese beiden Substanzen eines Substanzbehälters sind in bis zu der Entleerung voneinander getrennten Teilbereichen des Substanzbehälters aufgenommen.
Im Hinblick auf einen einzelnen Substanzbehälter ist dieser Substanzbehälter einheitlich mit einem einheitlichen Füllraum ausgebildet. Im Hinblick auf eine Befüllung der Vorrichtung mit Substanzbehältern ist diese Befüllung nur bei abgenommenem Deckel, also senkrecht zu einer Verlagerungsrichtung der Substanzbehälter in der Vorrichtung bei üblichem Betrieb der Vorrichtung möglich.
Bei einer aus der WO 02/053216 A2 bekannten Vorrichtung zum Inhalieren pulverförmiger Substanzen befinden sich die Substanzen in einer Kassette mit einzelnen Kammern. Aus der US 2010/294278 A1 ist gleichfalls eine Vorrichtung zum Inhalieren pulverförmiger Substanzen bekannt. Die Substanzen befinden sich in einzelnen Blisterpackungen, die wiederum in einem Blister-Drehteil aufgenommen sind. Zur Entleerung wird eine Blisterpackung aus dem Drehteil zunächst herausgeführt und dann aufgestochen. Aus der EP 2 115 595 A2 ist gleichfalls eine Vorrichtung zum Inhalieren pulverförmiger Substanzen bekannt, bei welcher in gleicher Weise in einer Kassette einzelne Blisterpackungen aufgenommen sind. Die Kassette wird durch ein schlittenförmiges Antriebsteil bewegt. In einer Entleerungsstellung einer Blisterpackung befindet sich die Blisterpackung in der Kassette.

### Zusammenfassung der Erfindung

Ausgehend von dem vorbeschriebenen Stand der Technik stellt sich der Erfindung die Aufgabe, einen vorteilhaften Substanzbehälter für eine solche Vorrichtung anzugeben.

Diese Aufgabe ist beim Gegenstand des Anspruches 1 gelöst, wobei darauf abgestellt ist, dass der Substanzbehälter zwei Teilbereiche aufweist, die jeweils gesondert eine Menge an Substanz aufweisen, und dass beide Teilbereiche eine öffenbare durchstechbare Abdeckung aufweisen.

Zufolge dieser Ausgestaltung ist ein neuartiger und vorteilhafter Substanzbehälter geschaffen. Dieser ermöglicht die Aufnahme von zwei Portionen einer pulverförmigen Substanz in demselben Substanzbehälter. Die Portionen können bezüglich ihrer Zusammensetzung unterschiedlich sein.

Besonders bei unterschiedlichen pharmazeutischen Substanzen, die zur Inhalation geeignet sind, jedoch zur Verbesserung der Wirkung erst unmittelbar im Zuge der Inhalation vermengt werden sollen, bietet sich ein solcher Substanzbehälter, dessen Teilbereiche zur Inhalation auch bevorzugt gleichzeitig öffenbar sind, an. Jede der unterschiedlichen Substanzen ist als Portion ist in einer gesonderten Kavität des Substanzbehälters aufgenommen. In vorteilhafter Weise können beide Teilbereiche jeweils eine öffenbare Abdeckung aufweisen. Ein solches Öffnen kann in der Vorrichtung durch eine Einstecheinrichtung erfolgen.

Auch bietet ein solcher Substanzbehälter die Möglichkeit bei entsprechender Ausbildung der Vorrichtung wahlweise im Zuge eines Inhalationsvorganges nur den einen oder anderen Teilbereich zu öffnen und mit der einsetzenden Luftströmung im Zuge der Inhalation zu räumen oder weiter alternativ beide Teilbereiche.

Hinsichtlich einer Vorrichtung kann vorteilhaft darauf abgestellt sein, dass der Substanzbehälter in der Entleerungsstellung sich in einer Aufnahmeausformung des Antriebsrads befindet.

Zufolge der vorgesehenen Lose-Anordnung einer Mehrzahl von Substanzbehältern in der Vorrichtung können in einer möglichen Ausgestaltung in der Vorrichtung Substanzbehälter mit beispielsweise unterschiedlichen Substanzen und/oder unterschiedlichen Dosierungen anordbar sein. So kann weiter beispielsweise die Dosierung von unmittelbar hintereinander angeordneten Substanzbehältern sukzessive ansteigen, beispielsweise bis auf ein medizinisch notwendiges Niveau, und gegebenenfalls mit den letzten Substanzbehältern in der Inhalierfolge sukzessiv wieder sinken.

Durch die vereinzelte Anordnung der Substanzbehälter ist eine individuelle Bestückung der Vorrichtung ermöglicht.

Die Substanzbehälter können sich in der Vorrichtung beziehungsweise in der Führungseinrichtung der Vorrichtung gegebenenfalls kettenartig bewegen, dies insbesondere bedingt zufolge der Aneinanderlage jeweils zweier in Verlagerungsrichtung unmittelbar aufeinanderfolgender Substanzbehälter. Eine insbesondere von außen durch den Benutzer ausgelöste Bewegung eines Substanzbehälters, beispielsweise in eine Entleerungsstellung, führt zufolge der Aneinanderlage zu einem sich über die Substanzbehälter fortpflanzenden Anlagedruck. Ein gegebenenfalls aktiv von außen, bevorzugt über das Antriebsrad unmittelbar in Bewegung versetzter Substanzbehälter kann quasi die gesamte weitere Reihe von Substanzbehältern vor sich herschieben.

Durch die eingefangene Stellung des Substanzbehälters in der Aufnahmeausformung des Antriebsrads ist eine vorgegebene und somit im Zuge einer Mehrzahl von Inhalationen jeweils definiert einnehmbare Entleerungsposition des Substanzbehälters erreichbar. Die Einnahme der Entleerungsposition ist dabei bevorzugt unabhängig von den in Transportrichtung nachrückenden und somit ansonsten einen Anlagedruck zum Verlagern des Substanzbehälters in der Führungseinrichtung aufbringenden Behältern. Vielmehr kann der in die Entleerungsposition verbrachte Substanzbehälter zur definierten Einnahme dieser Position - vorübergehend - aus der unmittelbaren Anlagedruck-Fortpflanzungskette ausgekoppelt sein.

Ein Verfahren zum Befüllen der Vorrichtung ist darauf abgestellt, dass in einem ersten Verfahrensschritt die Substanzbehälter bei bereits im Wesentlichen montierter Vorrichtung durch eine gesonderte Gehäuseöffnung in einen Vorratsraum der Vorrichtung eingebracht werden, dass in der Vorrichtung eine Führungseinrichtung für die Substanzbehälter in Form einer Führungsbahn ausgebildet ist und dass die Substanzbehälter in der Führungsbahn in einer auch bei üblicher Nutzung der Vorrichtung gegebenen Transportrichtung eingeführt werden und dass in einem zweiten Verfahrensschritt die gesonderte Gehäuseöffnung durch das ohne Zerstörung nicht mehr entfernbare Verschlussteil verschlossen wird.

Durch das vorgeschlagene Verfahren ist es ermöglicht, Herstellung und Montage der Vorrichtung einerseits sowie Befüllen der Vorrichtung andererseits räumlich und / oder zeitlich zu trennen. Die Vorrichtung kann gleichwohl mit Ausnahme des Verschlussteiles vollständig und funktionsbereit montiert werden. Die Substanzbehälter können hierbei sukzessive in eine mögliche Führungseinrichtung innerhalb der Vorrichtung eingeführt werden, beispielsweise mittels eines Schiebers, auf welchem oder in welchem in einer möglichen Ausgestaltung die Substanzbehälter in einer Hintereinanderanordnung vorgesehenen sein können.

Bezüglich der Vorrichtung zum Inhalieren pulverförmiger Substanzen, mit in der Vorrichtung aufgenommenen Substanzbehältern und einer Einstecheinrichtung, wobei die Einstecheinrichtung zur Öffnung des Substanzbehälters ausgebildet ist, weist der Substanzbehälter zwei Teilbereiche auf und ist in jedem Teilbereich eine Teilmenge der Substanz des Substanzbehälters enthalten. Darüber hinaus sind die Teilbereiche voneinander gesondert, jeder Teilbereich weist eine durch die Einstecheinrichtung öffenbare durchstechbare Abdeckung und die Teilmenge jeder der Teilbereiche ist nur durch die diesem Teilbereich zugeordnete durchstochene Abdeckung entleerbar.

Gemäß einer möglichen Ausgestaltung können die Substanzbehälter in der Führungseinrichtung der Vorrichtung eine endlose Reihe bilden. Wenngleich die Substanzbehälter bevorzugt untereinander unverbunden sind, ergibt sich bei einer solchen Ausgestaltung in der Vorrichtung eine endloskettenartige Anordnung der Substanzbehälter. Insbesondere kann die Führungseinrichtung nur mit einer an eine quer zu einer Bewegungsrichtung der Substanzbehälter in der Führungseinrichtung gegebenen Breite ausgebildet sein, so dass nur eine Hintereinanderanordnung - bezogen auf die Bewegungsrichtung - der Substanzbehälter in der Führungseinrichtung möglich ist. Die Substanzbehälter sind also einreihig hintereinander bevorzugt in der Vorrichtung aufgenommen und werden einreihig hintereinander in der Vorrichtung bevorzugt bewegt.

Bei der Bestückung der Vorrichtung mit den einzelnen Substanzbehältern kann der letzte einzuführende Substanzbehälter gemäß dem vorgeschlagenen Verfahren die vorbeschriebene Endloskette der Substanzbehälter quasi schließen. Der letzte Substanzbehälter kann schließlich dafür sorgen, dass bei einer Bewegung der Substanzbehälter-Kette der Anlagedruck sich über alle Substanzbehälter fortpflanzt. Dieser Anlagedruck kann gegebenenfalls sowohl in Richtung einer üblichen Verlagerungsrichtung der Substanzbehälter gegeben sein wie auch in einer hierzu entgegengesetzten Richtung.

Es kann ein Antriebselement vorgesehen sein, über das auf ein oder ausgewählte Substanzbehälter zur Bewegung der Substanzbehälter einwirkbar ist. Das Antriebselement kann unmittelbar oder auch mittelbar auf den einen oder die mehreren ausgewählten Substanzbehälter einwirken, so dass diese aktiv bewegt werden, während die weiteren Substanzbehälter in der Führungseinrichtung sich über den sich fortpflanzenden Anlagedruck unter den Substanzbehältern fortbewegen.

Dabei kann das Antriebselement als das vorbeschriebene Antriebsrad ausgebildet sein, mit radial offenen Aufnahmeausformungen, zur Einwirkung auf jeweils einen Substanzbehälter. Das Antriebsrad kann in Form eines Sternrades ausgebildet sein, aufweisend die vorbeschriebenen Aufnahmeausformungen.

In bevorzugter Ausgestaltung ist nur ein (Zahlwort) Antriebselement, insbesondere Antriebsrad, in der Vorrichtung vorgesehen, welches weiter bevorzugt zumindest auf den in die Entleerungsstellung zu bewegenden Substanzbehälter unmittelbar einwirkt. Darüber hinaus kann das Antriebsrad gegebenenfalls auch auf ein oder zwei, gegebenenfalls drei oder vier oder mehr, Substanzbehälter vor und/oder hinter dem in der Entleerungsstellung befindlichen Substanzbehälter einwirken (bezogen auf die Verlagerungsrichtung der Behälter).

In einer Aufnahmeausformung ist ein Substanzbehälter vollständig oder auch nur teilweise einfangbar. So kann die die Aufnahmeausformung begrenzende Wandung des Antriebsrades den eingefangenen Substanzbehälter um-fangsmäßig über einen Abschnitt umfassen, der es erlaubt, den Substanzbehälter zu führen und innerhalb der Führungseinrichtung zu leiten. So kann in einem Grundriss, in welchem sich die geometrische Drehachse des Antriebsrades bevorzugt als Punkt darstellt, die Aufnahmeausformung einen kreisabschnittförmigen Randkantenverlauf aufweisen, zur Zusammenwirkung mit einem beispielsweise kreiszylindrischen Abschnitt des Substanzbehälters.

Mit einem von dem Benutzer bewegbaren Antriebsteil kann das Antriebselement, insbesondere das Antriebsrad, in eine bevorzugt vorgegebene Transportrichtung bewegt werden. Eine Verlagerung des Antriebselementes entgegen der Transportrichtung ist bevorzugt unterbunden. Das Antriebsteil wird vom Benutzer willensbetont bewegt, insbesondere zur Verlagerung des Substanzbehältnisses in Richtung auf die Entleerungsstellung. In weiterer Ausgestaltung kann über das Antriebsteil auch eine Einstecheinrichtung derart verlagert werden, dass die Abdeckung des Substanzbehälters zum Öffnen des Substanzbehälters durchstoßen werden kann.

Dabei kann das Antriebselement koaxial und drehverbunden zu einem Betätigungsrad angeordnet sein. In diesem Zusammenhang kann weiter vorgesehen sein, dass das Antriebselement und das Betätigungsrad drehfest auf einer Antriebswelle angeordnet sind. Die geometrische Drehachse einer solchen Antriebswelle kann gemäß einer möglichen Ausgestaltung im Wesentlichen senkrecht ausgerichtet sein zur Bewegungsrichtung der Substanzbehälter in der Führungseinrichtung. Dabei kann weiter die Führungseinrichtung bahnartig die Antriebswelle umgebend angeordnet sein.

Mit dem Antriebsteil ist mittelbar oder unmittelbar über die Antriebswelle auf das Betätigungsrad einwirkbar. So kann von dem Benutzer über das Antriebsteil zugleich und bevorzugt in gleichem Maße auf das Antriebselement und das Betätigungsrad eingewirkt werden.

Das Betätigungsrad kann zur Verhinderung einer Rückdrehung, das heißt insbesondere eine Drehung entgegen der vorgegebenen Bewegungsrichtung der Substanzbehälter in der Führungseinrichtung, mit einer Rücklaufsperre zusammenwirken. So kann das Betätigungsrad beispielsweise ratschenartig mit einem abgefederten Blockierteil zusammenwirken, welches nur in einer Drehrichtung überlaufbar ist.

In handhabungstechnisch vorteilhafter Weise kann das Antriebsteil mit einer Verschlusskappe verbunden sein, so dass bei Bewegung der Verschlusskappe auf einem Verschwenkweg in eine Öffnungsstellung eine Bewegung des Antriebselements in Transportrichtung erfolgt. Hieraus ergibt sich in vorteilhafter Weise mit einer Vorbereitung der Vorrichtung zur Inhalation zufolge Verschwenken der Verschlusskappe in eine bevorzugt ein Mundstück der Vorrichtung freigebenden Öffnungsstellung zugleich eine Verlagerung der Substanzbehälter über das Antriebselement, womit in bevorzugter Ausgestaltung ein Substanzbehälter in die Entleerungsstellung verbracht wird. Es bedarf hierbei bevorzugt keiner zusätzlichen Handhabung. Vielmehr ist mit Verschwenken der Verschlusskappe in die Öffnungsstellung bevorzugt die Vorrichtung bezüglich der Positionierung des Substanzbehälters vorbereitet für die Inhalation.

Es kann das Bedürfnis bestehen, das Betätigungsrad bei der Bewegung der Verschlusskappe aus der Verschlussstellung in die Öffnungsstellung nur über einen Teil des Verschwenkweges über das Antriebsteil zu belasten. Dies insbesondere im Hinblick auf einen nur teilweise hierbei gegebenen Weg, den die Substanzbehälter zurücklegen sollen, wobei jedoch für die Betätigung der Einstecheinrichtung ein gegebenenfalls längerer Weg erforderlich ist. Das Antriebsteil kann hierzu nur über einen bevorzugt ersten Teil des Verschwenkweges auf das Betätigungsrad eingreifen und über den weiteren Teil des Verschwenkweges gegebenenfalls außer Eingriff stehend zu dem Betätigungsrad sein. Hierzu kann eine Kulissenführung vorgesehen sein, über welche das Antriebsteil im Zuge der Verschwenkbewegung der Verschlusskappe in die Eingriffsstellung zum Betätigungsrad oder aus dieser Eingriffstellung heraus gesteuert wird.

So kann diesbezüglich bevorzugt sein, dass mit Hilfe der Kulissenführung auf einem ersten Teil des Verschwenkweges das Antriebsteil in Eingriff mit der auch das Antriebselement antreibenden Antriebswelle und Betätigungsrad ist und auf einem zweiten Teilweg der Kappenbewegung außer Eingriff ist.

Bei einer Rückbewegung der Verschlusskappe in die Verschlussstellung kann das Antriebsteil wiederum durch die Kulissenführung vollständig außer Eingriff zu dem Betätigungsrad beziehungsweise zu der Antriebswelle über den gesamten nun rückwärts durchlaufenden Verschwenkweg, also den Rückweg, gesteuert sein.

So kann weiter das Antriebsteil ausfederbar ausgebildet sein. Über die Kulissenführung wird das Antriebsteil gegebenenfalls entgegen der sich einstellenden Rückstellkraft der Feder ausgelenkt oder aber auch unter Nutzung der federnden Rückstellkraft in einen sich ergebenden Kulissenbahnabschnitt gedrängt.

Die Federbarkeit kann gegeben sein zufolge federbarer Ausbildung eines Abschnitts des Antriebsteils.

In der Verschlussstellung der Verschlusskappe kann sich das Antriebsteil in einer Ausgangsstellung befinden, in welcher das Antriebsteil mit geringer oder fehlender Einfederung bevorzugt in der Kulissenführung einsitzt. Entsprechend ist in der unbenutzten Stellung der Vorrichtung, die einer bevorzugten Verwahrstellung der Vorrichtung entsprechen kann, die Feder des Antriebsteiles bevorzugt nicht oder nicht wesentlich hinsichtlich ihrer Federkraft belastet. Die für die Funktion der Vorrichtung gegebenenfalls notwendige Federbarkeit des Antriebsteils lässt auf Grund dieser bevorzugten Ausgestaltung auch bei Ausbildung des Antriebsteils mit dem ausfederbaren Abschnitt als Kunststoffteil nicht oder nicht wesentlich nach, so dass auch nach einem längeren Nichtgebrauch der Vorrichtung diese Funktion sicher ist.

Auch kann die Führungseinrichtung für die Substanzbehälter eine Führungsbahn aufweisen, die mit einer verschließbaren Einführöffnung für Substanzbehälter in dem Gehäuse in Verbindung steht. Die Führungsbahn gibt eine gerichtete Führung der Substanzbehälter vor. Hierbei können die Substanzbehälter mit Bezug auf die Verschieberichtung beidseitig in der Führungsbahn geführt sein.

Die verschließbare Einführöffnung kann gehäuseseitig vorgesehen sein, die in einer entsprechenden Offenstellung die Führungsbahn von außen zugänglich lässt. Über die Einführöffnung können Substanzbehälter in die Führungsbahn eingeführt werden, beispielsweise, wie auch bevorzugt, zur vollständigen Bestückung der Vorrichtung mit einer bevorzugt vorgegebenen Anzahl von Substanzbehältern.

So können in der Vorrichtung beispielsweise 15 bis 60 solcher Substanzbehälter in der Führungsbahn anordbar sein, weiter beispielsweise 20 bis 40, weiter insbesondere etwa 30 Substanzbehälter.

Die Einführöffnung kann durch ein nur zerstörend entfernbares Verschlussteil verschließbar sein. Nach Befüllen der Vorrichtung mit der vorgegebenen Anzahl an Substanzbehältern wird das Verschlussteil derart in die Einführöffnung eingesetzt, dass die Substanzbehälter gegen Herausfallen aus der Führungsbahn und aus der Vorrichtung insgesamt gesichert sind. Das Verschlussteil kann außenseitig, dem Benutzer zugewandt, optisch einen Teil des Vorrichtungsgehäuses darstellen.

Eine Entfernung des Verschlussteiles nach Einsetzen in die Einführöffnung ist bevorzugt nur durch vollständige oder teilweise Zerstörung des Verschlussteiles möglich wobei auch eine nur teilweise Zerstörung des Verschlussteiles insbesondere ein ordnungsgemäßes Wiedereinsetzen des Verschlussteiles in die Einführöffnung verhindert.

Eine entsprechende Festlegung des Verschlussteiles kann durch rastende Hintergriffabschnitte in Zusammenwirkung mit umgebenen Gehäusebereichen erreicht sein. Auch kann diesbezüglich eine Verklebung oder Verschweißung mit umgebenden Gehäuserandbereichen vorgesehen sein.

Auch kann, wie weiter auch bevorzugt, dass Verschlussteil innenseitig einen Teil der Führungsbahn bilden. So kann die dem Verschlussteil zugewandte Führungsbahn einen Teil der Führungsbahnwandung ausformen, gegebenenfalls, sofern führungsbahnseitig vorgesehen, einen Abschnitt weiterer Führungsmittel, wie beispielsweise Führungsstege oder dergleichen.

Darüber hinaus kann die Führungsbahn zugeordnet einer Einstecheinrichtung für einen Substanzbehälter Längsnuten aufweisen, in denen sich eventuell in der Einstecheinrichtung ausgetretene Substanz sammeln kann. Beispielsweise bei einer nicht durchgeführten Inhalation nach Aufstechen des in der Einstecheinrichtung befindlichen Substanzbehälters kann die Substanz gegebenenfalls in die Führungsbahn ausrieseln. Die Substanz wird bevorzugt in den Längsnuten der Führungsbahn gesammelt, so dass die Verlagerbarkeit der Substanzbehälter auf der Führungsbahn nicht beeinträchtigt wird.

Hierzu kann der Boden und / oder die Decke der Führungsbahn entsprechend nutenartige Vertiefungen gegenüber der Anlage- beziehungsweise Gleitfläche für die Substanzbehälter aufweisen.

Die Längsnuten können sich hierbei über die gesamte Strecke der Führungsbahn erstrecken, wobei gegebenenfalls mehrere Längsnuten in Nebeneinanderanordnung vorgesehen sein können. Auch können diese mehreren Längsnuten über [im Wesentlichen] quer zur Längserstreckung der Führungsbahn verlaufende Quernuten untereinander verbunden sein, um so eine günstige Verteilung etwaig ausgerieselter Substanz in den Nuten zu erreichen.

Darüber hinaus können über die Führungsnuten Substanzpartikel auch gezielt in einen Bereich weitergeleitet werden, in dem ein bevorzugt gesondert zu der Führungsbahn ausgebildeter Sammelraum ausgebildet ist. In diesem kann die in den Längsnuten transportierte Substanz gegebenenfalls aufgefangen werden. Damit kann erreicht werden, dass die Substanz letztlich gegebenenfalls vollständig aus dem Bereich der Führungsbahn heraus verbracht ist.

Der Substanzbehälter kann im Wesentlichen zylinderförmig gebildet sein, weiter bevorzugt kreiszylinderförmig.

Die beiden Teilbereiche des Substanzbehälters können bei einer solchen Ausgestaltung jeweils endseitig, mit Bezug auf eine Längserstreckungsachse des zylinderförmigen Substanzbehälters, vorgesehen sein, so dass diese in Erstreckungsrichtung der Achse betrachtet gegenüberliegend angeordnet sein können.

Die Teilbereiche des Substanzbehälters können über einen sich im Wesentlichen in einer Querebene zur Längsachse erstreckenden Boden voneinander getrennt sein.

Um eine günstige Räumung der Teilbereiche (Kavitäten) durch die Luftströmung zu erreichen, können die Teilbereiche jeweils halbschalenartig geformt sein, so dass sich bevorzugt keine strömungsmäßigen Todbereiche einstellen. Die Teilbereiche können hierzu einen durchgehend gekrümmt ausgebildeten Bodenbereich ausweisen. Diese durchgehende Krümmung ist bevorzugt auch gleich in allen möglichen in Richtung der Längsachse gegebenen Querschnitten gegeben.

In diesem Zusammenhang erweist es sich von Vorteil, wenn der Substanzbehälter bezogen auf eine Zylinderlängsachse etwa mittig eine außen umlaufende, quer zur Zylinderachse ausgerichtete Nut aufweist. Zufolge der Ausbildung einer solchen Nut ergeben sich insbesondere im Zusammenhang mit der halbschalenförmigen Ausgestaltung der Teilbereiche und mit Bezug auf einen Längsschnitt durch den Substanzbehälter, in welchem Längsschnitt sich die Längsachse als Linie darstellt, annähernd gleiche Wandungsstärken des Behälters auch im mittigen, dem die Teilbereiche zueinander trennenden Boden aufweisenden Bereich. Insbesondere bei Substanzbehältern, die als KunststoffSpritzteile hergestellt sind, können sich bei örtlich hohen (zu hohen) Wandstärken, wie sich diese im mittleren Bereich einstellen würden ohne die vorgesehene Nut, gegebenenfalls Einfallerscheinungen zeigen. Darüber hinaus ergibt sich eine Material- und Gewichtsersparnis.

So besteht weiter der Substanzbehälter bevorzugt aus einem Hartkunststoff, wie beispielsweise Polypropylen oder auch Polyethylen.

Die an dem Substanzbehälter gegebenenfalls vorgesehene wandungsaußenseitige und sich in Umfangsrichtung erstreckende Nut kann darüber hinaus weiter auch zur Führung des Substanzbehälters in der Führungsbahn genutzt sein, beispielsweise zufolge Eingriffs eines Führungssteges in die Nut, ein- oder beidseitig des Substanzbehälters mit Bezug auf einen Grundriss, in welchem sich die Längsachse als Punkt darstellt.

Die den jeweiligen Teilbereich des Substanzbehälters überdeckende und durchstoßbare Abdeckung kann, wie auch bevorzugt, aus einer Folie bestehen, beispielsweise einer Aluminiumfolie. Hierbei kann die Folie mit einem Stirnrand des Substanzbehälters beziehungsweise mit einem den Teilbereich umgebenden Rand verschweißt sein. Insbesondere kommt hierbei bevorzugt Ultraschallschweißen zur Anwendung.

Dabei kann der diesbezügliche Stirnrand des Substanzbehälters vor der Verschweißung der Folie vereinzelte Stege, beispielsweise mit Bezug auf die Längsachse radial ausgerichtete Stege aufweisen, die im Zuge des Schweißvorganges schmelzen und sich unter Vergleichmäßigung über den gesamten Stirnwand mit der Folie verbinden.

Die in der Kavität zu bevorratende portionierte Substanz wird selbstverständlich vor Anschweißen der Folie zur Überdeckung der Kavität eingebracht.

Die Substanzbehälter werden über die Einführöffnung in die Führungsbahn eingeführt, dies in einer auch bei der üblichen Nutzung der Vorrichtung gegebenen Bewegungsrichtung, so dass eine gegebenenfalls vorgesehene Rücklaufsperre die Einführung nicht hindert.

### Kurze Beschreibung der Zeichnungen

Nachstehend ist die Erfindung anhand der beigefügten Zeichnung erläutert, die aber lediglich Ausführungsbeispiele darstellt. Ein Teil, das nur bezogen auf eines der Ausführungsbeispiele erläutert ist und bei einem weiteren Ausführungsbeispiel aufgrund der dort herausgestellten Besonderheit nicht durch ein anderes Teil ersetzt ist, ist damit auch für dieses weitere Ausführungsbeispiel als jedenfalls mögliches vorhandenes Teil beschrieben. Die Zeichnung zeigt:
- Fig. 1: eine Vorrichtung zum Inhalieren pulverförmiger Substanzen in perspektivischer Darstellung, betreffend die verschlossene Nichtgebrauchsstellung;
- Fig. 2: die Vorrichtung gemäß Figur 1 in einer weiteren perspektivischen Darstellung;
- Fig. 3: die Herausvergrößerung des Bereiches III vor Verschließen einer gehäuseseitigen Einführöffnung durch ein Verschlussteil;
- Fig. 4: in perspektivischer Einzeldarstellung einen Substanzbehälter für die Vorrichtung;
- Fig. 5: die Draufsicht gemäß Pfeil V in Figur 4;
- Fig. 6: die Ansicht gemäß Pfeil VI in Figur 4 gegen den Substanzbehälter;
- Fig. 7: den Schnitt gemäß der Linie VII-VII in Figur 6;
- Fig. 8: die Herausvergrößerung des Bereiches VIII in Figur 7, bei an dem Substanzbehälter festgelegter Abdeckung;
- Fig. 9: eine der Figur 8 im Wesentlichen entsprechende Detailschnittdarstellung, jedoch betreffend eine Situation vor Festlegung der Abdeckung an dem Substanzbehälter;
- Fig. 10: eine perspektivische Detaildarstellung des Substanzbehälters, partiell geschnitten, betreffend die Situation gemäß Figur 9;
- Fig. 11: eine explosionsperspektivische Darstellung der Vorrichtung;
- Fig. 12: eine vergrößerte explosionsperspektivische Darstellung des Bereiches XII in Figur 11;
- Fig. 13: die vergrößerte explosionsperspektivische Darstellung des Bereiches XIII in Figur 11;
- Fig. 14: die Vorrichtung in einer weiteren explosionsperspektivischen Darstellung;
- Fig. 15: eine vergrößerte explosionsperspektivische Darstellung des Bereiches XV in Figur 14;
- Fig. 16: eine explosionsperspektivische Darstellung des Bereiches XVI in Figur 14;
- Fig. 17: in perspektivischer Darstellung die Anordnung eines Antriebsritzels, eines Antriebselementes und eines drehfest auf einer Antriebswelle angeordneten Betätigungsrades, weiter eines Übertragungszahnrades und eines Zählrades;
- Fig. 18: in perspektivischer Explosionsdarstellung das Zählrad und das Übertragungsrad sowie den das Zählrad und das Übertragungszahnrad aufnehmenden Gehäuseabschnitt;

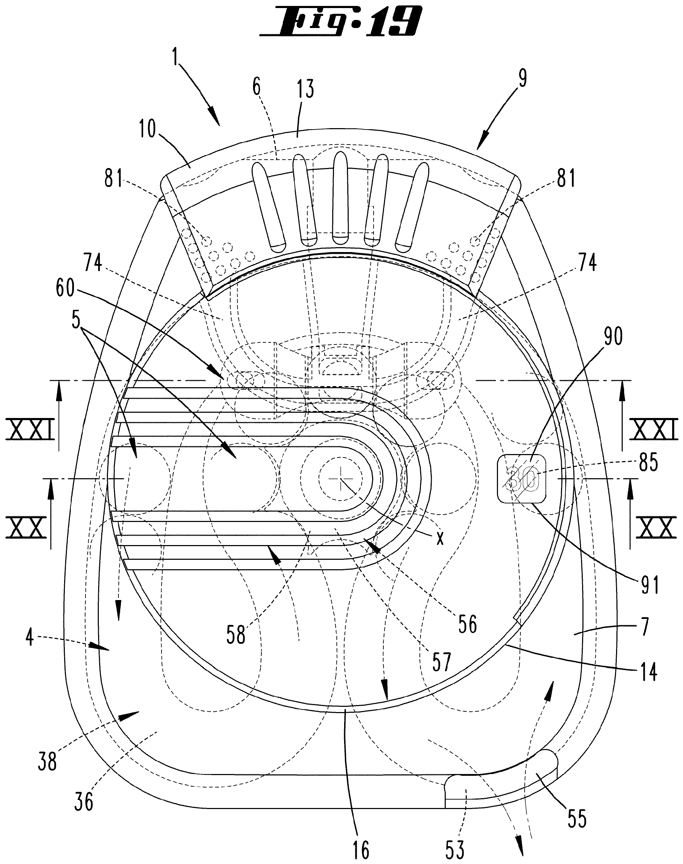

- Fig. 19: die Vorrichtung in Draufsicht, betreffend die verschlossene Nichtgebrauchsstellung;
- Fig. 20: den Schnitt gemäß der Linie XX-XX in Figur 19;

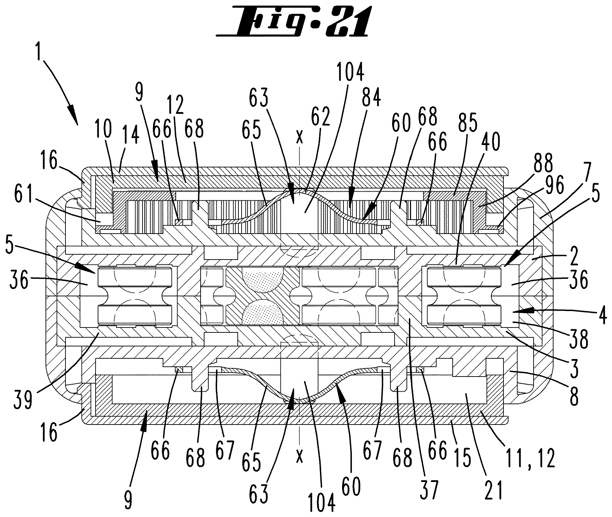

- Fig. 21: den Schnitt gemäß der Linie XXI-XXI in Figur 19;
- Fig. 22: den Schnitt gemäß der Linie XXII-XXII in Figur 20;
- Fig. 23: eine der Figur 22 entsprechende Darstellung, jedoch ohne in der Vorrichtung aufnehmbaren Substanzbehältern;
- Fig. 23a: eine der Figur 23 entsprechende Darstellung, jedoch eine alternative Ausführungsform betreffend;
- Fig. 24: eine weitere der Figur 22 entsprechende Darstellung, betreffend eine Situation im Zuge eines Befüllens der Vorrichtung mit Substanzbehältern;

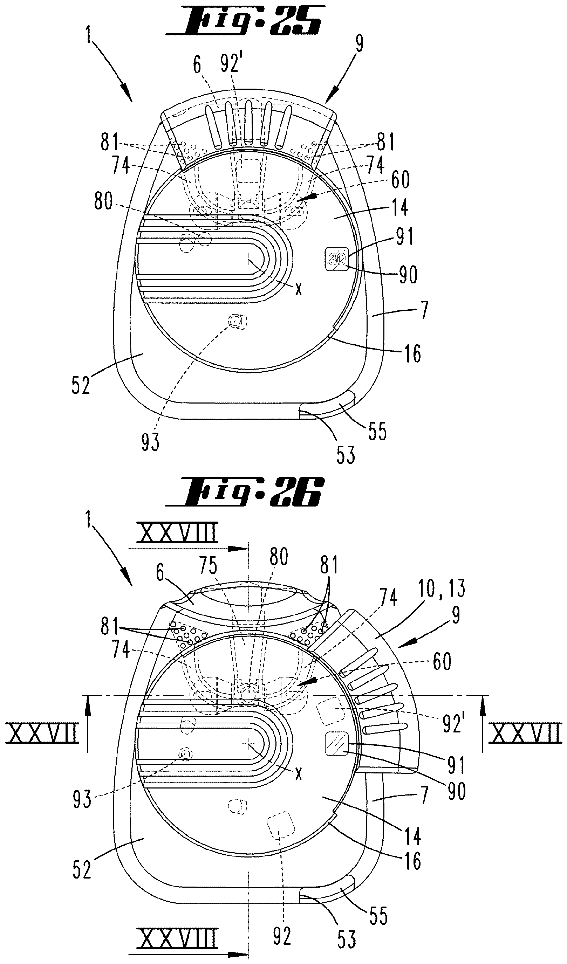

- Fig. 25: eine im Wesentlichen der Figur 19 entsprechende Draufsichtdarstellung der Vorrichtung;
- Fig. 26: eine der Figur 25 entsprechende Darstellung, jedoch im Zuge einer Schwenkbewegung einer Verschlusskappe in Richtung auf eine Öffnungsstellung;

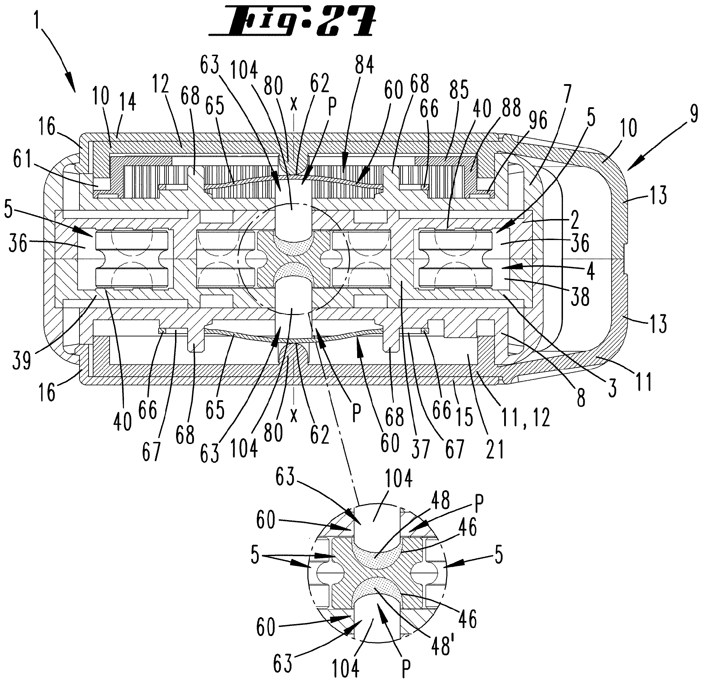

- Fig. 27: den Schnitt gemäß der Linie XXVII-XXVII in Figur 26;

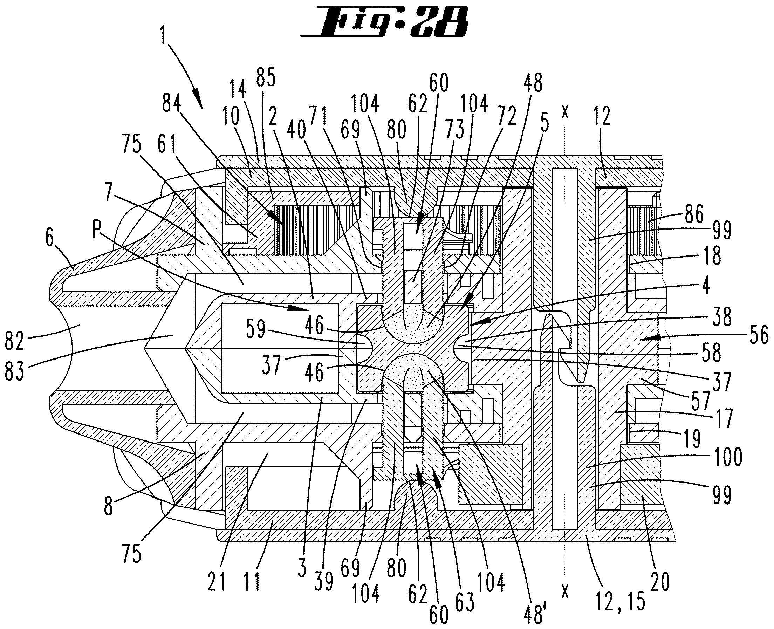

- Fig. 28: den Schnitt gemäß der Linie XXVIII-XXVIII in Figur 26;

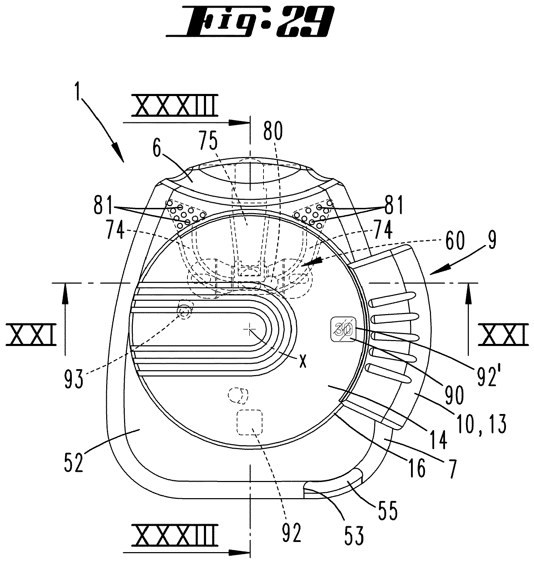

- Fig. 29: eine Folgedarstellung zur Figur 26, die Verschlusskappen-Offenstellung betreffend;

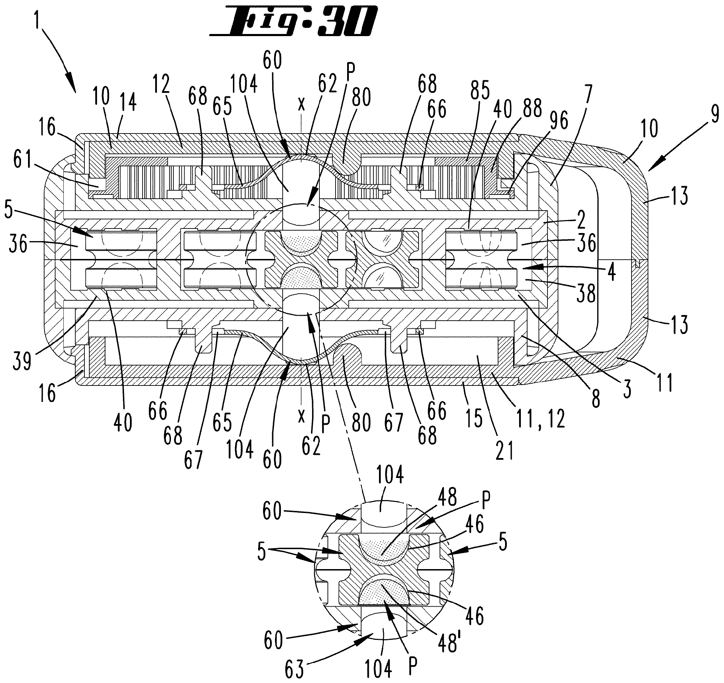

- Fig. 30: den Schnitt gemäß der Linie XXX-XXX in Figur 29;
- Fig. 31: die Vorrichtung in perspektivischer Darstellung, die Verschlusskappen-Offenstellung und somit die Inhalation-Bereitschaftsstellung betreffend;
- Fig. 32: eine im Wesentlichen der Figur 31 entsprechende Darstellung, jedoch partiell aufgebrochen;
- Fig. 32a: eine der Figur 32 entsprechende Darstellung, eine alternative Ausführungsform betreffend;
- Fig. 33: den Schnitt gemäß der Linie XXXIII-XXXIII in Figur 29;

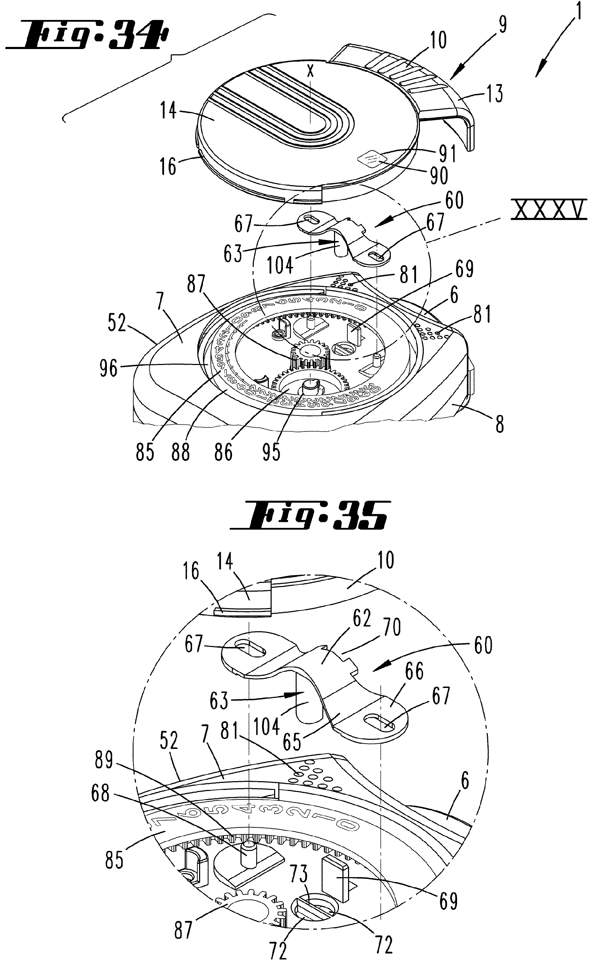

- Fig. 34: eine teilweise explosionsperspektivische Darstellung der Vorrichtung, betreffend den Bereich einer Einstecheinrichtung;
- Fig. 35: die vergrößerte Darstellung des Bereiches XXXV in Figur 34;
- Fig. 36: die Einstecheinrichtung in einer perspektivischen Einzeldarstellung;
- Fig. 36a: die Einstechrichtung in einer zweiten Ausführungsform;
- Fig. 36b: die Herausvergrößerung des Bereichs XXXVIb in Figur 36a;
- Fig. 37: die Einstecheinrichtung in einer Seitenansicht;
- Fig. 38: eine Schnittdarstellung betreffend die Anordnung von zwei Einstecheinrichtungen in der Vorrichtung zum Durchstoßen zweier Abdeckungen eines Substanzbehälters;
- Fig. 38a: eine Schnittdarstellung gemäß Fig. 38, jedoch die Ausführung gemäß Fig. 36a betreffend;
- Fig. 39: den Schnitt gemäß der Linie XXXIX-XXXIX in Figur 37;
- Fig. 39a: die Schnittdarstellung gemäß Figur 39, die zweite Ausführung betreffend;
- Fig. 40: eine der Figur 37 im Wesentlichen entsprechende Seitenansichtsdarstellung, jedoch betreffend die Anordnung von zwei Einstecheinrichtungen;
- Fig. 41: eine perspektivische Schnittdarstellung durch einen Substanzbehälter, bei von einer Einstecheinrichtung gemäß Fig. 36 durchstoßenen Abdeckungen;
- Fig. 41a: eine der Figur 41 entsprechende Darstellung, jedoch bei durch eine Einstechrichtung gemäß Figur 36a durchstoßenen Abdeckungen;
- Fig. 42: eine im Wesentlichen schematische Draufsichtdarstellung auf die Vorrichtung, betreffend das getriebeartige Zusammenwirken des Zählwerks und des Antriebselementes zum Verbringen eines Substanzbehälters in eine Entleerungsposition sowie die Beaufschlagung des Einstechmittels in Abhängigkeit von der Schwenkverlagerung der Verschlusskappe, betreffend die Kappenschließstellung;
- Fig. 43: eine Folgedarstellung zu Figur 42, im Zuge der Verschwenkbewegung der Verschlusskappe in Richtung auf die Offenstellung;

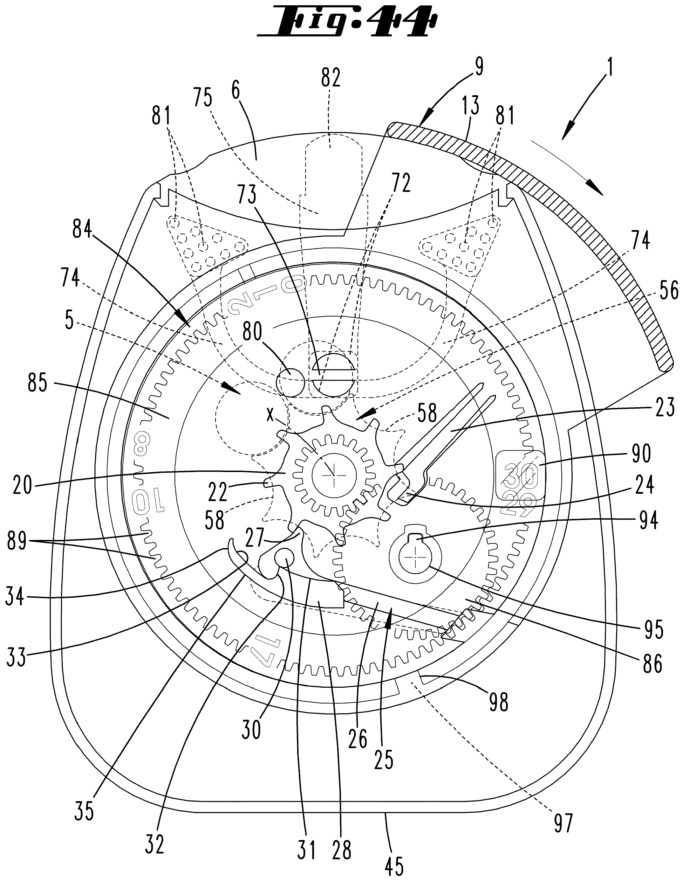

- Fig. 44: eine Folgedarstellung zur Figur 43;

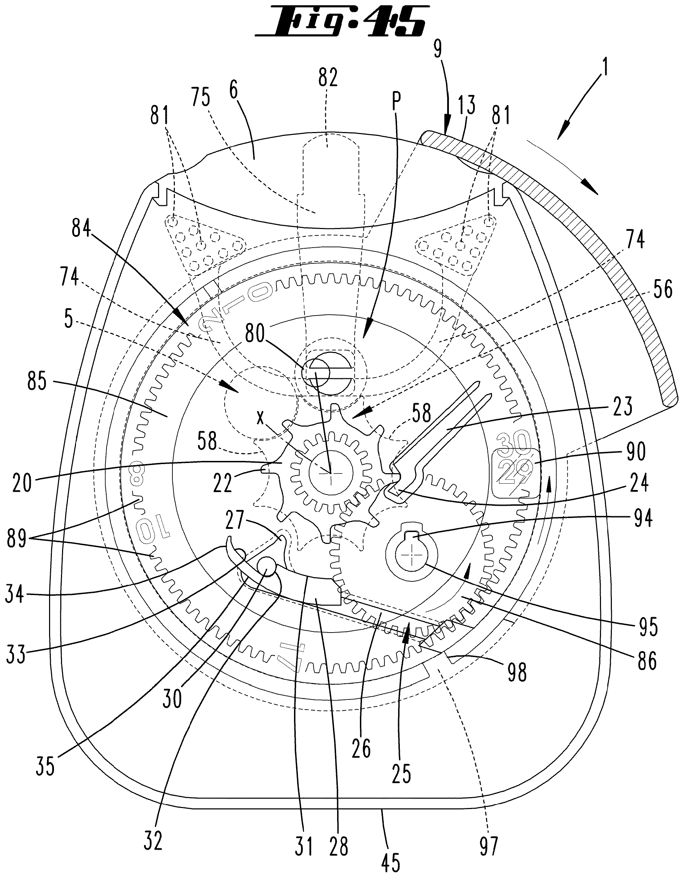

- Fig. 45: eine Folgedarstellung zur Figur 44, betreffend eine Zwischenstellung, in welcher der Substanzbehälter die Entleerungsposition erreicht hat;

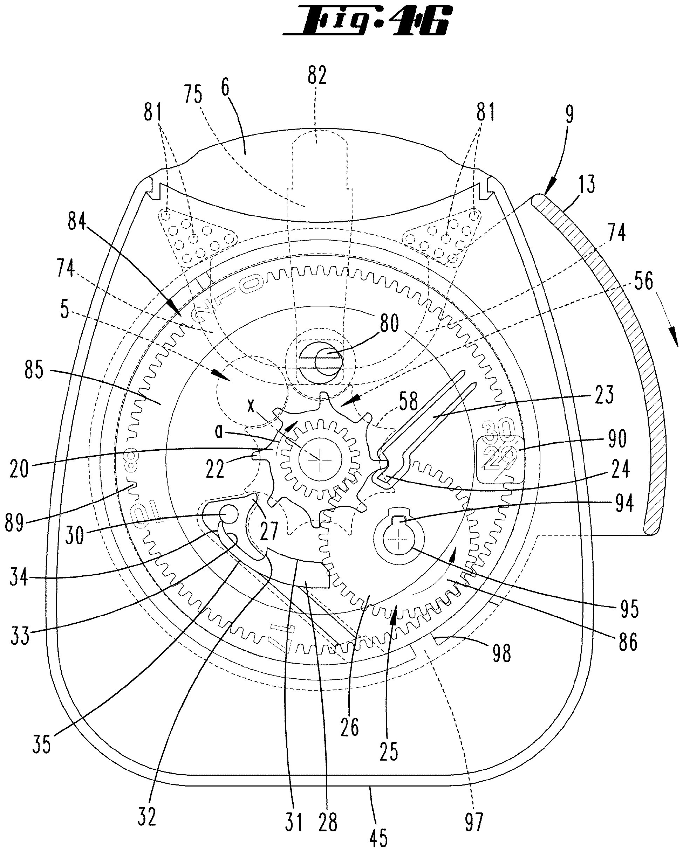

- Fig. 46: eine weitere Folgedarstellung im Zuge der Verschwenkung der Verschlusskappe in die Offenstellung, betreffend die Beaufschlagungssituation des Einstechmittels;
- Fig. 47: eine weitere Folgedarstellung bei Erreichen der Verschlusskappen-Offenstellung;
- Fig. 48: in schematischer Darstellung den Bereich der Luftkanäle gemäß XLVIII in Figur 47;
- Fig. 48a: eine der Figur 48 entsprechende Darstellung, jedoch die Ausführungsform gemäß Figur 32a betreffend;

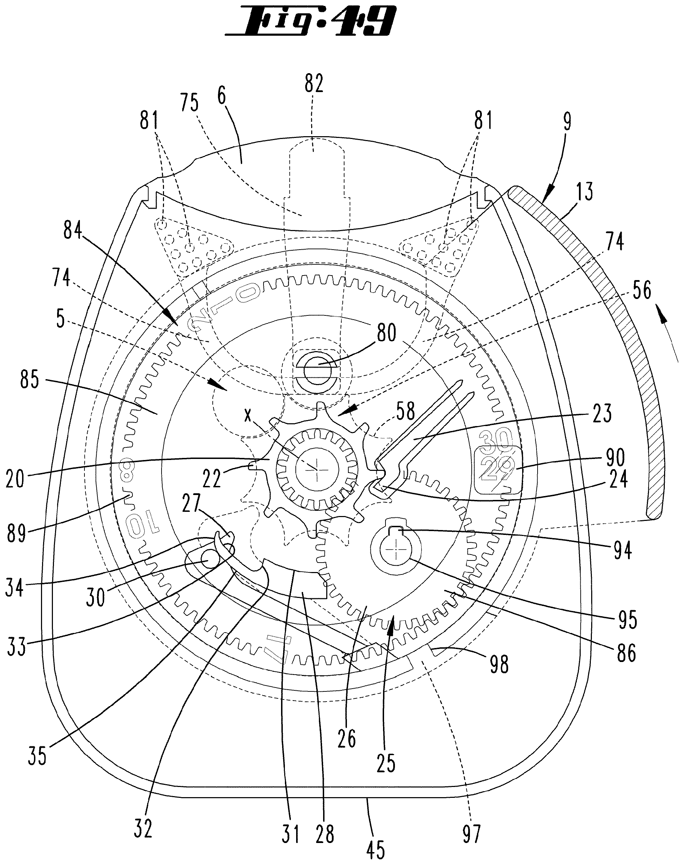

- Fig. 49: eine Zwischenstellung im Zuge der Verschwenkung der Verschlusskappe aus der Offenstellung in Richtung auf die Verschlussstellung;

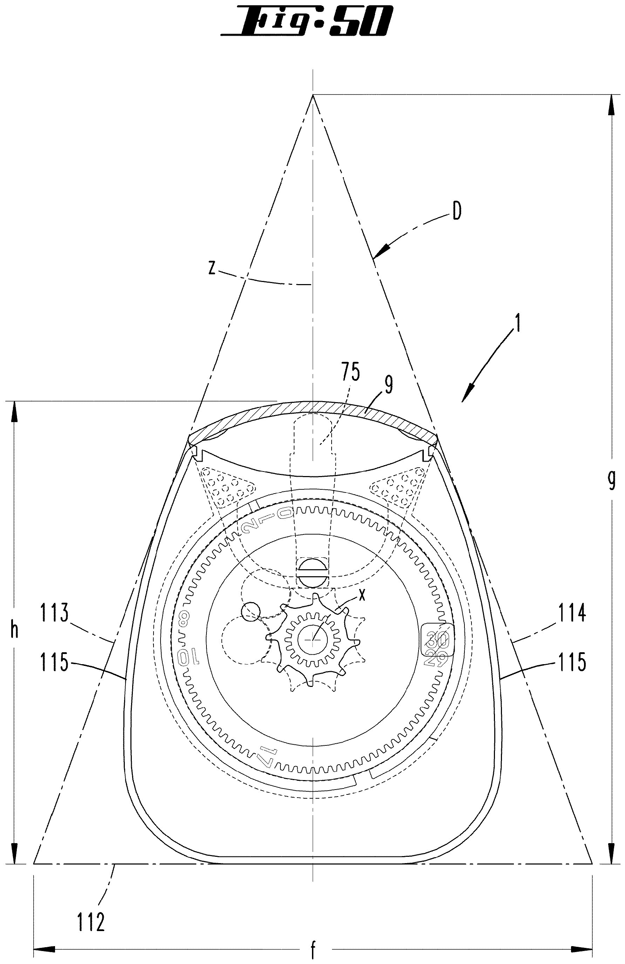

- Fig. 50: eine schematische Darstellung der Vorrichtung in Draufsicht, zur Erläuterung einer geometrischen Grundkontur der Vorrichtung und deren Abmessungen

### Beschreibung der Ausführungsformen

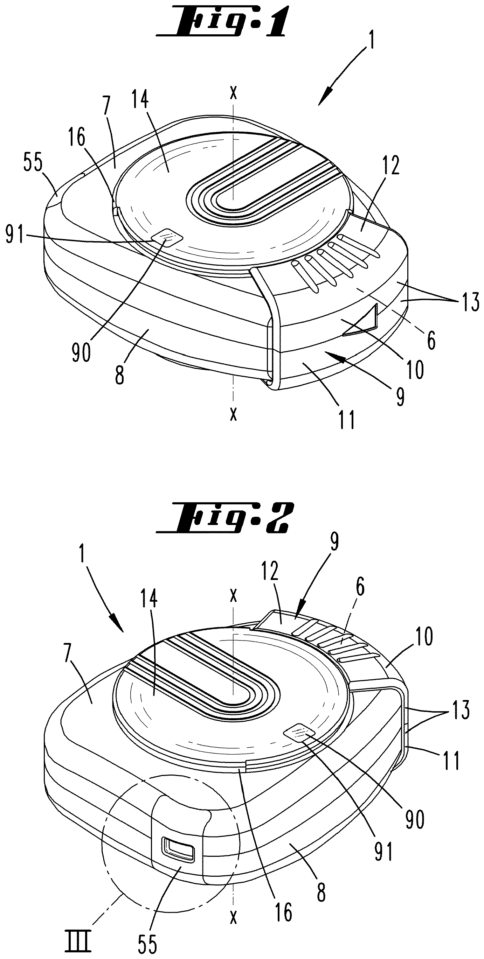

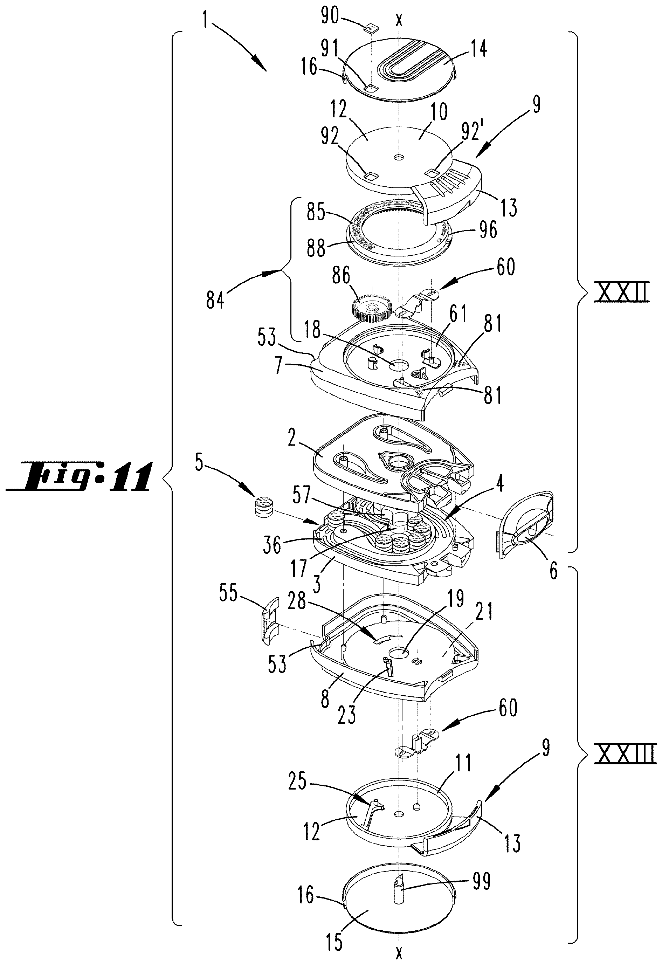

Dargestellt und beschrieben ist, zunächst mit Bezug zu den Figuren 1 und 2 sowie 11., eine Vorrichtung 1 zum Inhalieren pulverförmiger Substanzen 48, 48'. Die Vorrichtung 1 weist bevorzugt ein Mundstück 6 auf, weiter eine Einstecheinrichtung 60 mit Einstechmitteln 63, zur Öffnung eines Substanzbehälters 5, wobei bevorzugt eine Mehrzahl von sukzessiv in eine Entleerungsposition P bewegbare Substanzbehälter 5 vorgesehen sind, die untereinander unverbunden in einer vorrichtungsfesten Führungseinrichtung 4 zur unmittelbaren Anlage aneinander aufgenommen sind. Zur Zählung und Anzeige der durchgeführten oder noch verbleibenden Inhalationsvorgänge ist ein Zählwerk 84 vorgesehen.

Die nachstehend aufgeführten wesentlichen Bestandteile der Vorrichtung 1 können, wie bevorzugt, aus einem Kunststoff, insbesondere Hartkunststoff wie Polypropylen oder Polyethylen bestehen.

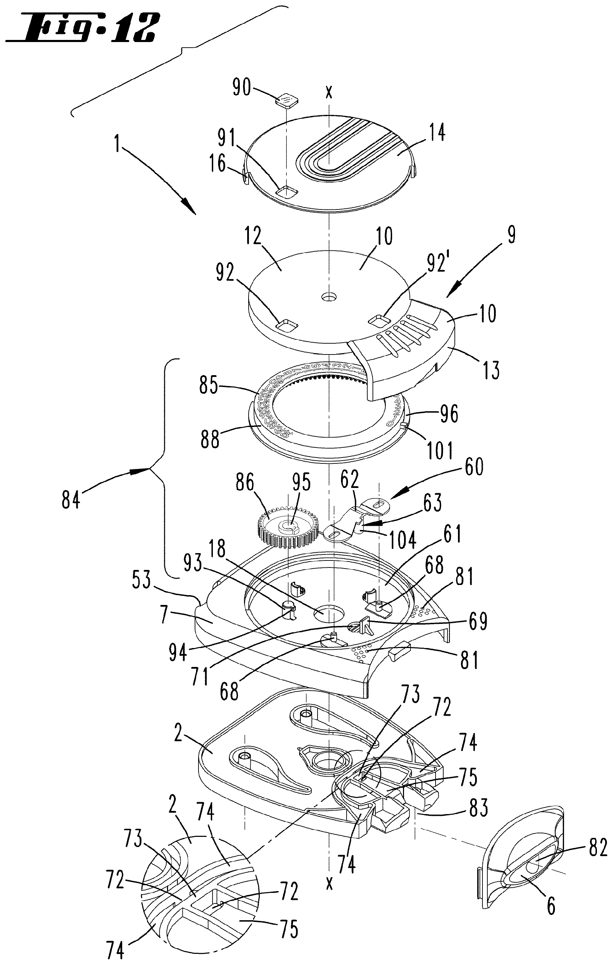

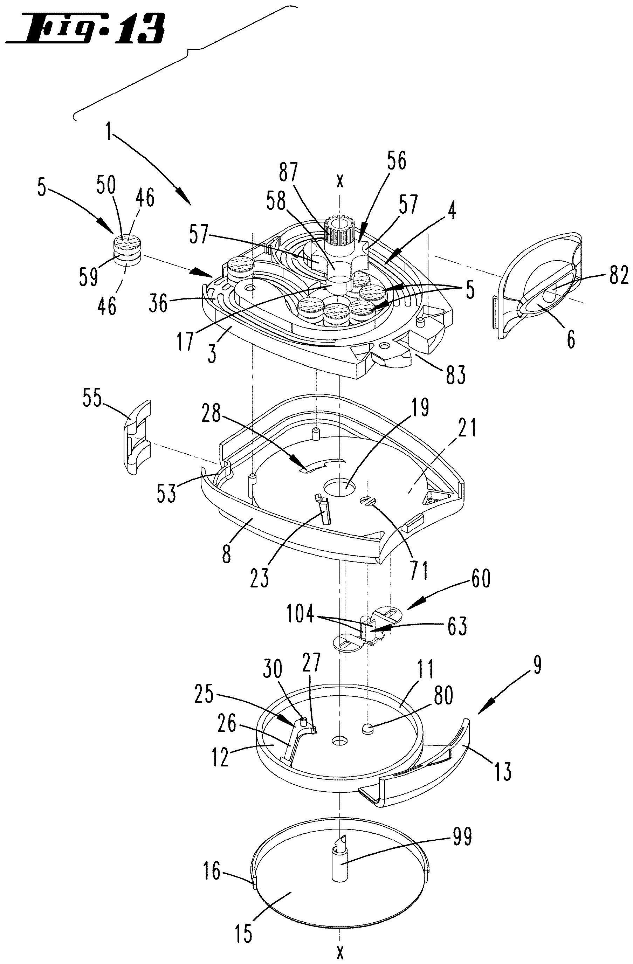

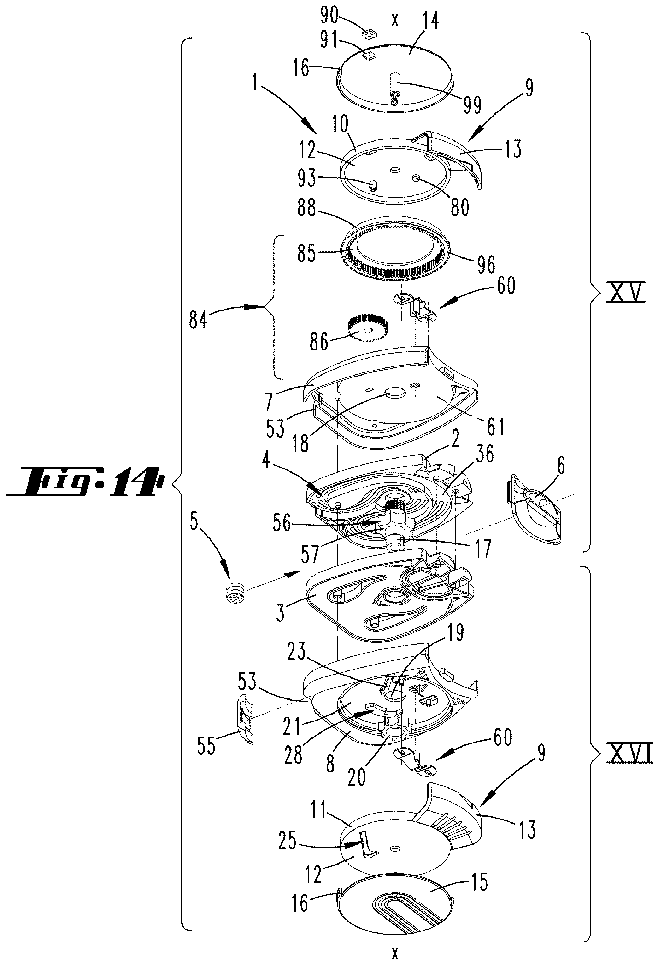

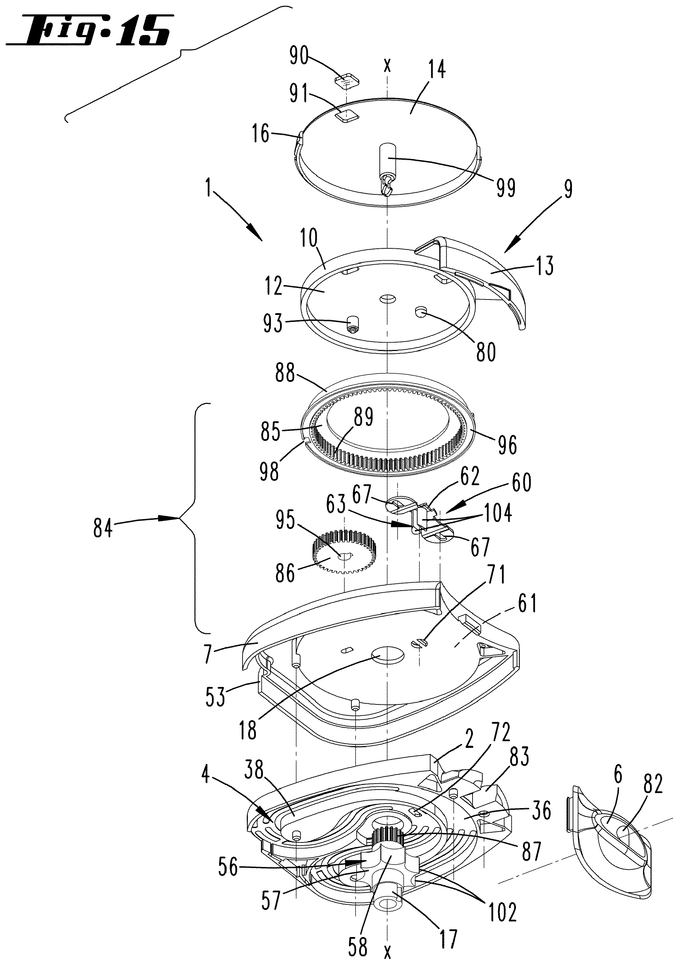

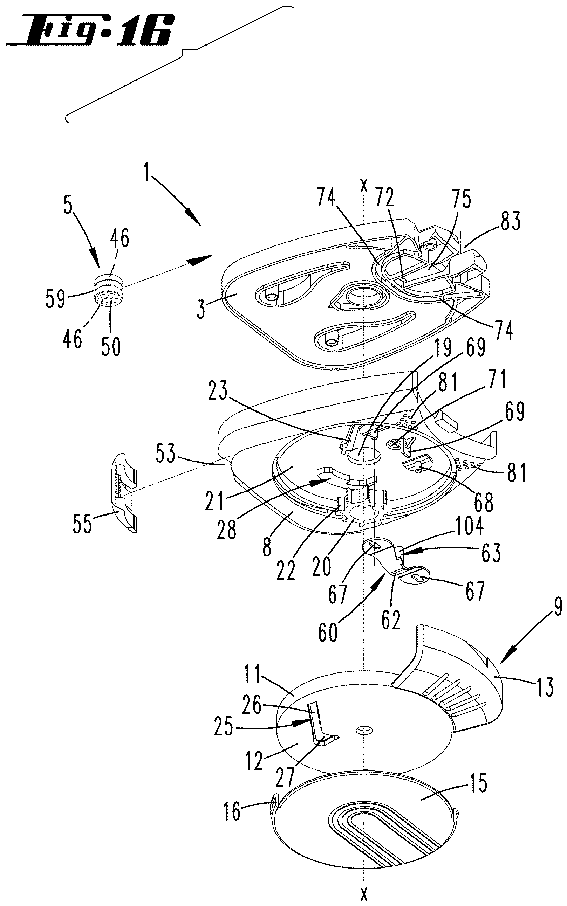

Wie insbesondere aus den explosionsperspektivischen Darstellungen in den Figuren 12 bis 17 ersichtlich, kann die Vorrichtung 1 im Wesentlichen zunächst aus einem Gehäuse-Innenoberteil 2 und einem Gehäuse-Innenunterteil 3 bestehen, die unmittelbar aneinander anliegend zwischen sich die Führungseinrichtung 4 für die Substanzbehälter 5 belassen.

An diesen Gehäuse-Innenteilen 2 und 3 ist ein Mundstück 6 angeordnet, über welches zufolge Einatmen der Inhalationsvorgang durchgeführt werden kann.

Weiter ist Bestandteil der Vorrichtung 1 sowohl ein Gehäuse-Schalenoberteil 7 als auch ein Gehäuse-Schalenunterteil 8, die im Wesentlichen entlang aufeinander zugewandter Randkanten aneinanderliegend zwischen sich die Gehäuse-Innenteile aufnehmen.

Gehäuse-Schalenoberteil 7 und Gehäuse-Schalenunterteil 8 bilden im Wesentlichen zusammen mit dem Mundstück 6 die äußere Kontur der Vorrichtung 1.

Auch ist im Wesentlichen Bestandteil der Vorrichtung 1 eine Verschlusskappe 9 für das Mundstück 6. Die Verschlusskappe 9 ist im Wesentlichen gebildet aus zwei, jeweils dem Gehäuse-Schalenoberteil 7 und dem Gehäuse-Schalenunterteil 8 zugeordneten Verschlusskappenteilen, nämlich einem Verschlusskappen-Oberteil 10 und einem Verschlusskappen-Unterteil 11.

Jedes Verschlusskappenteil weist hierbei einen tellerartigen Deckabschnitt 12 auf und einen von diesem Deckabschnitt 12 nach außen abragenden, in einem Querschnitt L-förmig gebildeten Kappenabschnitt 13.

Entlang der aufeinander zuweisenden freien Randkanten der L-förmigen Kappenabschnitte 13 können diese miteinander verbunden sein, beispielsweise zufolge einer Verklebung oder Verschweißung, so dass sich insgesamt diesbezüglich eine im Querschnitt im Wesentlichen U-förmige Kappe zum Überfangen des Mundstückes 6 ergibt.

Die Deckenabschnitte 12 sind in paralleler Ausrichtung zueinander vorgesehen und relativ zu den Gehäuse-Innenteilen und den Gehäuse-Schalenteilen um eine geometrische Schwenkachse x schwenkverlagerbar.

Das Verschlusskappen-Oberteil 10 kann darüber hinaus von einem tellerartigen Deckelteil 14 überfangen und das Verschlusskappen-Unterteil 11 von einem ebenfalls bevorzugt tellerartigen Bodenteil 15 unterfangen sein.

Die bezüglich des vorbeschriebenen Gehäuses benutzten Begrifflichkeiten "ober" und "unter" beziehungsweise "Boden" oder "Deckel" beziehen sich allein auf die zeichnerischen Darstellungen beispielsweise in den Figuren 12 bis 17. Es ergibt sich bei der vorgeschlagenen Vorrichtung 1 bevorzugt keine bevorzugte Ausrichtung der Vorrichtung zur Erlangung einer korrekten Handhabung. So kann beispielsweise die Oberseite bei der Nutzung durchaus auch die Unterseite der Vorrichtung 1 bilden.

Bodenteil 15 und Deckelteil 14 können, wie auch dargestellt, jeweils einen teilweise um die Schwenkachse x umlaufenden Kragen 16 aufweisen. Deren in Umfangsrichtung weisenden freien Stirnrandkanten können jeweils einen Schwenkanschlag für die Verschlusskappe 9 in der Verschlusskappen-Schließstellung und in der Verschlusskappen-Offenstellung bieten. Entsprechend ist der Schwenkwinkel der Verschlusskappe 9 begrenzt, so beispielsweise auf einen Schwenkwinkel von etwa 50 bis 70 Grad, bevorzugt etwa 60 Grad.

Entlang der geometrischen Schwenkachse x ausgerichtet ist in der Vorrichtung 1 eine Antriebswelle 17 vorgesehen. Diese kann, wie auch bevorzugt, relativ zu der Verschlusskappe 9 und dem Gehäuse 52 schwenk- beziehungsweise drehverlagerbar sein. Eine Führung kann die Antriebswelle 17 im Bereich angepasster Bohrungen 18, 19 im Gehäuse-Innenoberteil 2 und Gehäuse-Innenunterteil 3 beziehungsweise in dem Gehäuse-Schalenoberteil 7 und dem Gehäuse-Schalenunterteil 8 erfahren.

Auch kann die Antriebswelle 17 auf einem relativ zu der Antriebswelle 17 feststehenden Achskörper 100 aufgenommen sein. Der Achskörper 100 kann dabei gebildet sein aus jeweils an dem Deckelteil 14 und an dem Bodenteil 15 zentral angeformten und aufeinanderzuweisenden Hohlzapfen 99. Die Hohlzapfen 99 können, wie auch bevorzugt, miteinander verrastet sein, derart, dass das hierdurch insgesamt rastverschlossene Gehäuse 52 bevorzugt nicht mehr zerstörungsfrei öffenbar ist.

Die Antriebswelle 17 ist über die Verschlusskappe 9 ratschenartig antreibbar, derart, dass zufolge einer Schwenkverlagerung der Verschlusskappe 9, insbesondere aus einer das Mundstück 6 verschließenden Grundstellung heraus in eine Offenstellung, die Antriebswelle 17 um ein vorgegebenes Winkelmaß drehverlagert wird. Eine Rückschwenkverlagerung der Verschlusskappe 9 aus der Offenstellung in die Schließstellung bewirkt bevorzugt keine Drehmitnahme der Antriebswelle 17.

Hierzu ist die Antriebswelle 17 mit einem Betätigungsrad 20 drehfest verbunden. Dieses sitzt in dem dargestellten Ausführungsbeispiel in einer muldenartigen Vertiefung 21 des Gehäuse-Schalenunterteils 8 zwischen diesem Schalenunterteil und dem Verschlusskappen-Unterteil 11.

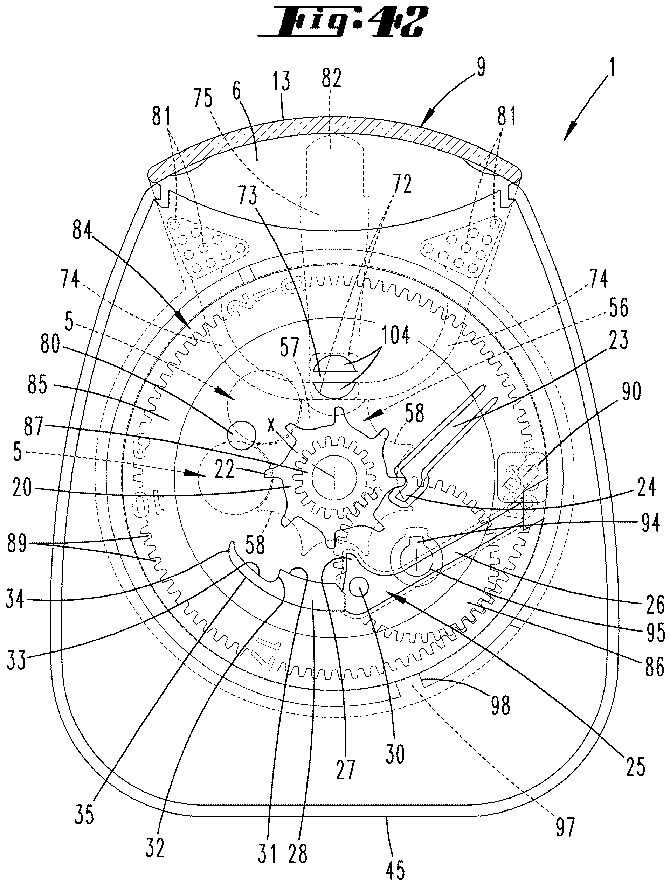

Das Betätigungsrad 20 kann, wie auch dargestellt, über im Wesentlichen radial vorstehende Mitnahmevorsprünge 22 verfügen. In dem dargestellten Ausführungsbeispiel sind über den Umfang gleichmäßig verteilt acht solcher Mitnahmevorsprünge 22 vorgesehen, wobei jeder Mitnahmevorsprung 22 abweichend von einer strengen Radialen zur geometrischen Schwenkachse x einen spitzen Winkel von etwa 20 bis 30 Grad einschließen kann, so dass eine mit Bezug auf eine Draufsicht beispielsweise gemäß Figur 42 gedachte Mittenlinie durch einen Mitnahmevorsprung 22 das Betätigungsrad 20 sekantenartig schneiden kann.

Das Betätigungsrad 20 wirkt im Bereich der Vertiefung 21 mit einer Rücklaufsperre 23 zusammen. Diese kann, wie auch dargestellt, als federnder Abschnitt des Gehäuse-Schalenunterteiles 8, einstückig mit diesem ausgebildet sein.

Die Rücklaufsperre 23 ist mit einer Rastnase 24 versehen, zur Zusammenwirkung mit den Mitnahmevorsprüngen 22 des Betätigungsrades 20, wobei die Rastnase 24 weiter so ausgebildet sein kann, dass diese durch die Mitnahmevorsprünge 22 nur in der vorgegebenen Drehrichtung a des Betätigungsrades 20 überlaufen werden kann. Bei einem solchen Überlaufen federt die Rastnase 24 entsprechend aus.

Entgegen der Drehrichtung a ist ein solches Überlaufen nicht möglich. Vielmehr blockiert die Rastnase 24 der Rücklaufsperre 23 eine Drehung des Betätigungsrades 20 in diese Richtung.

Das Betätigungsrad 20 und über dieses die Antriebswelle 17 ist drehbewegbar zufolge Einwirkung eines Antriebsteiles 25 auf einen der Mitnahmevorsprünge 22 des Betätigungsrades 20. Das Antriebsteil 25 kann, wie auch bevorzugt, Teil der Verschlusskappe 9, insbesondere Teil des Verschlusskappen-Unterteils 11 sein.

Das Antriebsteil 25 kann dabei einstückig und materialeinheitlich mit dem diesbezüglichen Verschlusskappen-Unterteil 11 ausgebildet sein. Hierbei kann weiter das Antriebsteil 25 einen an dem Deckabschnitt 12 wurzelnden Federarm 26 ausweisen, der endseitig eine vorsprungartige Mitnahmenase 27 ausformt. Diese Mitnahmenase 27 ist geeignet zur Zusammenwirkung mit einem Mitnahmevorsprung 22 des Betätigungsrades 20.

Zufolge der Anordnung der Mitnahmenase 27 an einem Federarm 26 ist die Mitnahmenase 27 im Wesentlichen quer zur Längserstreckung des Federarmes 26 federnd ausweichbar gestaltet.

In einer Grundstellung des Antriebsteiles 25, die der Mundstück-Verschlussstellung der Verschlusskappe 9 beispielsweise gemäß Figur 42 entspricht, liegt das Antriebsteil 25 unbelastet und bevorzugt ohne Einfederung in der Vertiefung 21 ein. Erst mit Verschwenken der Verschlusskappe 9 aus der Verschlussstellung in Richtung auf die Offenstellung erfolgt eine gesteuerte Verlagerung des Antriebsteiles 25 entgegen einer sich hierbei einstellenden Rückstellkraft im Bereich des Federarmes 26.

Diese Steuerung ist erreicht über eine relativ zu dem Antriebsteil 25 und dem Betätigungsrad 20 feststehende Kulissenführung 28. Diese kann, wie auch dargestellt, bodenseitig der Vertiefung 21 vorgesehen sein. Die Kulissenführung 28 kann darüber hinaus als eine im Wesentlichen konzentrisch zur Schwenkachse x verlaufende, in Umfangsrichtung gestufte Rippe ausgebildet sein, wobei diese zunächst eine Steuerfläche 29 ausweist, zur Zusammenwirkung mit einem an dem Antriebsteil 25 im Bereich der Mitnahmenase 27 angeformten Steuerzapfen 30.

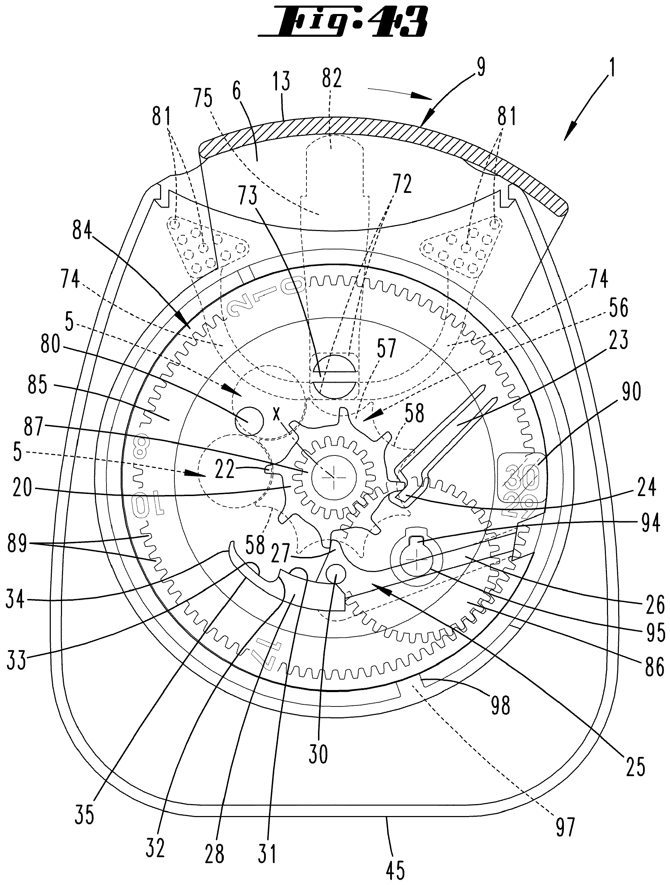

Mit Verschwenken der Verschlusskappe 9 aus der Verschlussstellung gemäß Figur 42 heraus tritt der Steuerzapfen 30 zunächst gegen die Steuerfläche 29, wobei unter weiterer Schwenkverlagerung der Verschlusskappe 9 der Steuerzapfen 30 und hierüber die Mitnahmenase 27 mit Bezug zu der Schwenkachse x nach radial innen gesteuert wird, dies unter Aufbau einer Rückstellkraft im Federarm 26 (vergleiche Figur 43).

Die Mitnahmenase 27 tritt in eine Lücke umfangsmäßig zwischen zwei Mitnahmevorsprünge 22 des Betätigungsrades 20.

Die im Zuge der weiteren Schwenkverlagerung der Verschlusskappe 9 in Richtung auf die Offenstellung entlang einer konzentrisch zur Schwenkachse x verlaufenden ersten Anlagefläche 31 geführte Mitnahmenase 27 schleppt in Anlage an einem Mitnahmevorsprung 22 das Betätigungsrad 20 in Drehrichtung a mit, dies über einen vorbestimmten Winkelbereich, der es ermöglicht, einen Substanzbehälter 5 in eine Entleerungsposition P zu verbringen.

Der über das Antriebsteil 25 bewirkte Drehwinkel des Betätigungsrades 20 ist bevorzugt geringer als der mögliche, anschlagbegrenzte Verschwenkwinkel der Verschlusskappe 9.

Die im Zuge dieser weiteren Schwenkverlagerung der Verschlusskappe 9 über den Federarm 26 mitgeschleppte Mitnahmenase 27 fällt über einen stufenartigen Rücksprung 32 in der Kulissenführung 28 auf eine relativ zu der Schwenkachse x gegenüber der ersten Anlagefläche 31 radial nach außen versetzte zweite Anlagefläche 33. Dieser Abfall der Mitnahmenase 27 ist unterstützt durch die Rückstellkraft des Federarmes 26. Die Mitnahmenase 27 verlässt den Zusammenwirkungsbereich mit dem Mitnahmevorsprung 22 des Betätigungsrades 20, so dass die weitere Schwenkverlagerung der Verschlusskappe 9 keinen weiteren Dreheinfluss auf das Betätigungsrad 20 bewirkt.

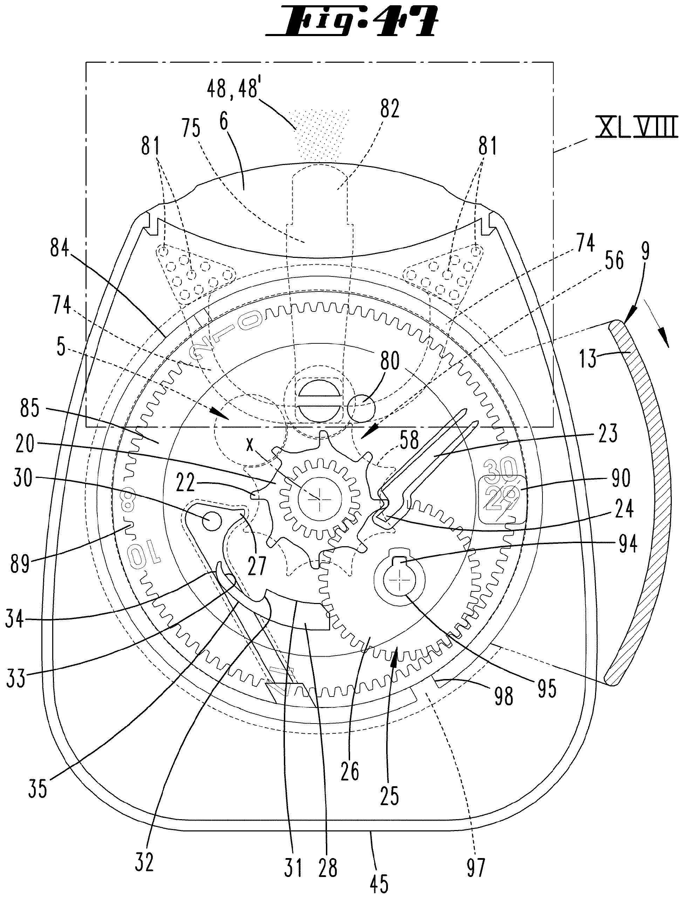

Mit Erreichen der Verschlusskappen-Offenstellung gemäß Figur 47 verlässt der Steuerzapfen 30 die Kulissenführung 28, wonach das Antriebsteil 25 erneut eine bezüglich der Federkräfte entspannte Position einnehmen kann.

Die stufenartige Ausgestaltung der Kulissenführung 28 bewirkt weiter, dass nach einer vollständigen Drehverlagerung des Betätigungsrades 20 um den vorgegebenen Drehwinkel und somit nach Verlagerung eines Substanzbehälters 5 in die Entleerungsposition P zwingend die weitere Verlagerung der Verschlusskappe 9 in Richtung auf die bevorzugt anschlagbegrenzte Offenstellung erfolgen muss. Eine Rückverlagerung der Verschlusskappe 9 kann erst erfolgen, nachdem die Kulissenführung 28 mit Erreichen der Kappen-Offenstellung verlassen wurde (gemäß der Stellung in Figur 47).

Aus dieser Verschlussklappen-Offenstellung heraus wird - bevorzugt nach einem zuvor durchgeführten Inhalationsvorgang - zufolge Verlagerung der Verschlusskappe 9 in Richtung auf die Grundstellung beziehungsweise in Richtung auf die Mundstück-Verschlussstellung gemäß Figur 42 der Steuerzapfen 30 des Antriebsteiles 25 gegen eine endseitige weitere Steuerfläche 34 bewegt, die eine ausfedernde Verlagerung des Steuerzapfens 30 und somit der Mitnahmenase 27 mit Bezug zu der Schwenkachse x nach radial außen bewirkt, so dass der Steuerzapfen 30 hiernach im Zuge der weiteren Schwenkverlagerung der Verschlusskappe 9 in Richtung auf die Mundstück-Verschlussstellung entlang einer gegenüber der ersten und der zweiten Anlagefläche 31, 33 wiederum gegenüber der Schwenkachse x radial nach außen beabstandeten dritten Anlagefläche 35 bewegt wird. Hierbei ergibt sich eine gegenüber der Vorverlagerung des Antriebsteiles 25 entgegengesetzt wirkende Feder-Rückstellkraft im Bereich des Federarmes 26, bis der Steuerzapfen 30 kurz vor Erreichen der Verschlusskappen-Endstellung die Kulissenführung 28 verlässt und die federunbelastete Stellung gemäß Figur 42 wieder einnimmt.

Die zwischen beziehungsweise durch die Gehäuse-Innenober- und Unterteile 2, 3 gebildete Führungseinrichtung 4 bildet einen Vorratsraum 36 für eine Mehrzahl von nicht untereinander verbundenen Substanzbehältern 5. Gehäuse-Innenoberteil 2 und Gehäuse-Innenunterteil 3 bilden die Führungseinrichtung 4 beziehungsweise den Vorratsraum 36 in Erstreckungsrichtung der Schwenkachse x betrachtet jeweils etwa hälftig. Hierbei kann sich je Gehäuse-Innenteil mit Bezug auf einen Querschnitt, in welchem sich die Schwenkachse x als Linie darstellt, ein U-förmiger Abschnitt ergeben, wobei die U-Öffnungen aufeinander zuweisen und die diese U-Öffnung begrenzenden Seitenwandungen 37 an den Stirnflächen aneinander liegen.

Es ergib sich so eine mit Bezug auf die Schwenkachse x ober- und unterseitig wie auch seitlich durch die Seitenwände 37 begrenzte Führungsbahn 38, bei welcher die Seitenwände 37 quer zur Längserstreckung der Führungsbahn 38 bevorzugt angepasst an einen Außendurchmesser d der Substanzbehälter 5 zueinander beabstandet sind.

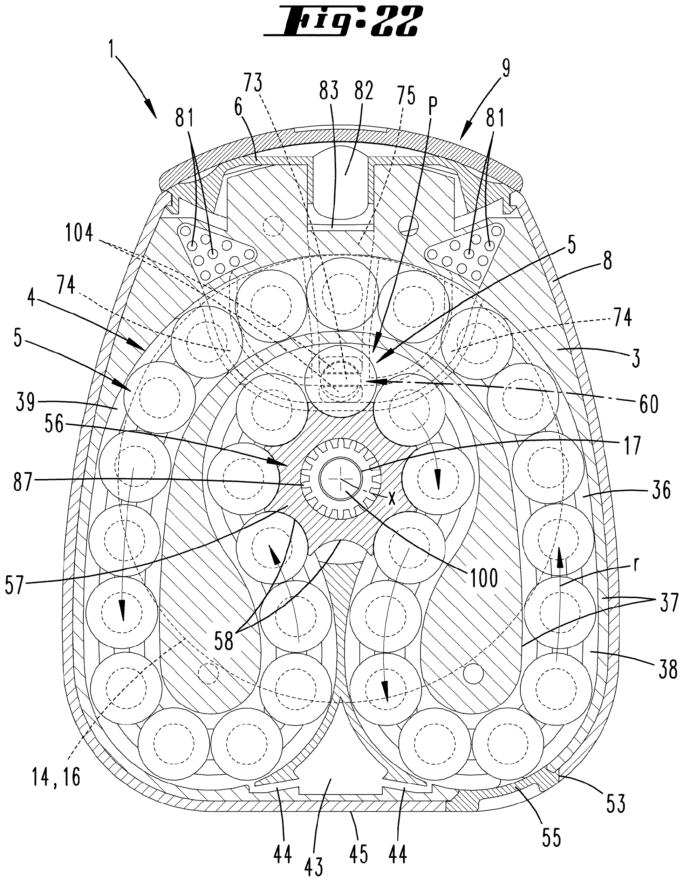

Die Führungsbahn 38 ist, wie beispielsweise aus Figur 22 ersichtlich, mäanderförmig in Art einer Endlosbahn in der Vorrichtung 1 vorgesehen, hierbei im Bereich der Schwenkachse x eine konzentrisch zur Schwenkachse x verlaufende Schleife bilden.

Von dieser Schleife ausgehend erstreckt sich die Führungsbahn 38 beidseitig der Schleife zunächst in Richtung einer dem Mundstück 6 abgewandten Rückseite der Vorrichtung 1, um hiernach sich jeweils über einen nach außen gerichteten Bogen zurück in Richtung des das Mundstück 6 aufweisenden Vorderbereiches der Vorrichtung 1 zu erstrecken. Ein die Schleife umfassender, gegebenenfalls konzentrisch zur Schwenkachse x verlaufender Bogen verbindet die Bahnabschnitte zu einer dadurch gekrümmten Endlosbahn, die bevorzugt keine gerade gestreckt verlaufende Abschnitte aufweist.

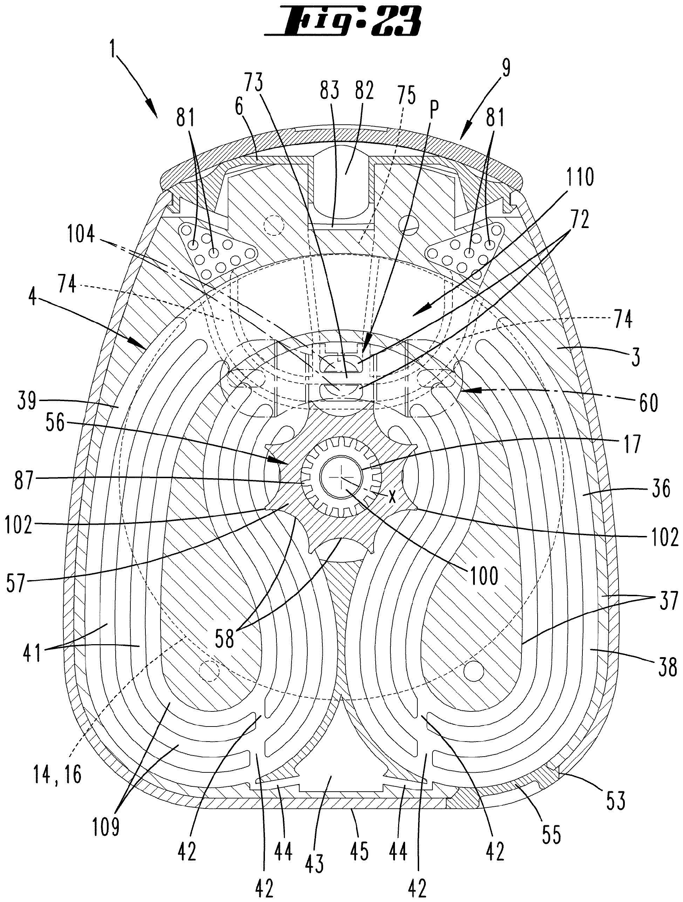

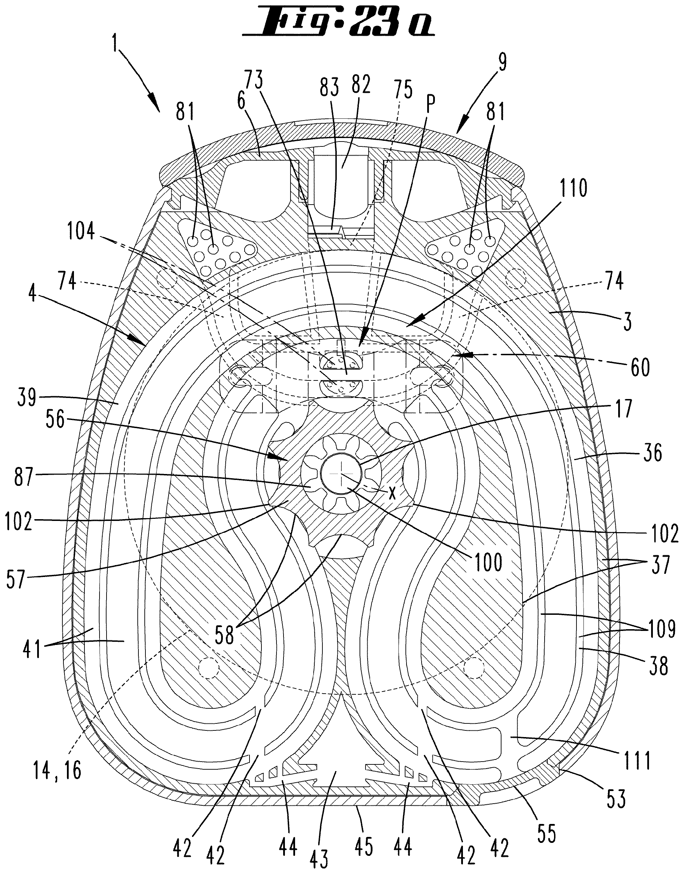

Im Bereich des Bahnbodens 39 und/oder der Bahndecke 40 können, wie auch bevorzugt, über die gesamte oder einen Großteil der Führungsbahn 38 Längsnuten 41 vorgesehen sein. So können beispielsweise diesbezüglich zwei oder drei Längsnuten 41 vorgesehen sein, die sich in Längserstreckungsrichtung der Führungsbahn 38 erstrecken und hierbei zueinander quer zur Längserstreckung beabstandet sind (vergleiche Figur 23).

Auch können diese Längsnuten 41 bereichsweise über Quernuten 42 verbunden sein.

Gemäß beispielsweise der Darstellung in Figur 23 können Längsstege 109, die sich ebenfalls in Längsrichtung erstrecken, die Längsnuten 41 voneinander in der Längsrichtung trennen. In einem dem Mundstück 6 zugeordneten Zenitbereich 110 der Führungseinrichtung 4 können die Längsstege 109 nicht durchlaufend beziehungsweise vorher auslaufend ausgebildet sein. Die Längsnuten 41 können in diesem Bereich entsprechend frei über die gesamte quer zur Längserstreckung der Längsnuten 41 betrachtete Breite in den Bahnboden 39 einlaufen. Es sind in diesem Bereich somit keine zu unterscheidenden Längsnuten gegeben.

Bei dem in Figur 23a gezeigten Ausführungsbeispiel hingegen ist auch der Zenitbereich 110 mit die Längsnuten 41 trennenden Längsstegen 109 durchsetzt.

Auch können gemäß Figur 23a die Längsstege 109 über einen Brückenabschnitt 111, bevorzugt im Bereich einer Einführ- bzw. Gehäuseöffnung 53, untereinander in Querrichtung zur Längserstreckung der Stege verbunden sein.

Über diese Nutstruktur im Boden- und/oder Deckenbereich der Führungsbahn 38 kann in günstiger Weise gegebenenfalls aus einem Substanzbehälter 5 austretende Substanz 48, 48', insbesondere pulverförmige Substanz 48, 48', in die durch die Nuten gebildeten Zwischenräume geführt werden und gegebenenfalls über diese in einen weiter vorgesehenen Sammelraum 43. Dieser Sammelraum 43 kann sich in einem mit Bezug auf das Mundstück 6 rückseitigen Zwickelbereich zwischen den Wendebereichen der Führungsbahn 38 ergeben.

Der Sammelraum 43 ist in diesem Fall bevorzugt über Stichwege 44 mit den zugewandten Abschnitten der Führungsbahn 38 verbunden.

Die diesbezügliche Rückseite der Vorrichtung 1 kann eine Stellfläche 45 für die Vorrichtung 1 anbieten, so dass in diesem Fall der Sammelraum 43 in einen untersten Bereich der Vorrichtung 1 angeordnet ist und die gegebenenfalls sich in den Nuten 41 und 42 ansammelnde Substanz schwerkraftbedingt in Richtung Sammelraum 43 gelangt.

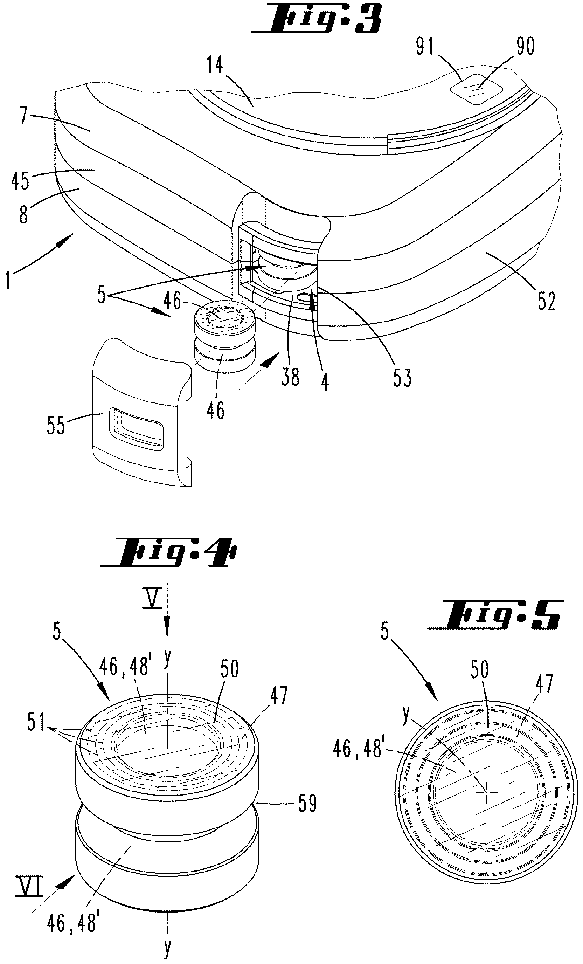

Der insbesondere in den Figuren 4 bis 10 dargestellte Substanzbehälter 5 kann zunächst und im Wesentlichen kreiszylindrisch geformt sein, aufweisend eine Zylinderachse y, die in der Aufnahmestellung des Substanzbehälters 5 in der Vorrichtung 1 beziehungsweise in der Führungseinrichtung 4 gleichgerichtet ausgerichtet ist zu der geometrischen Schwenkachse y beziehungsweise der Drehachse der Antriebswelle 17.

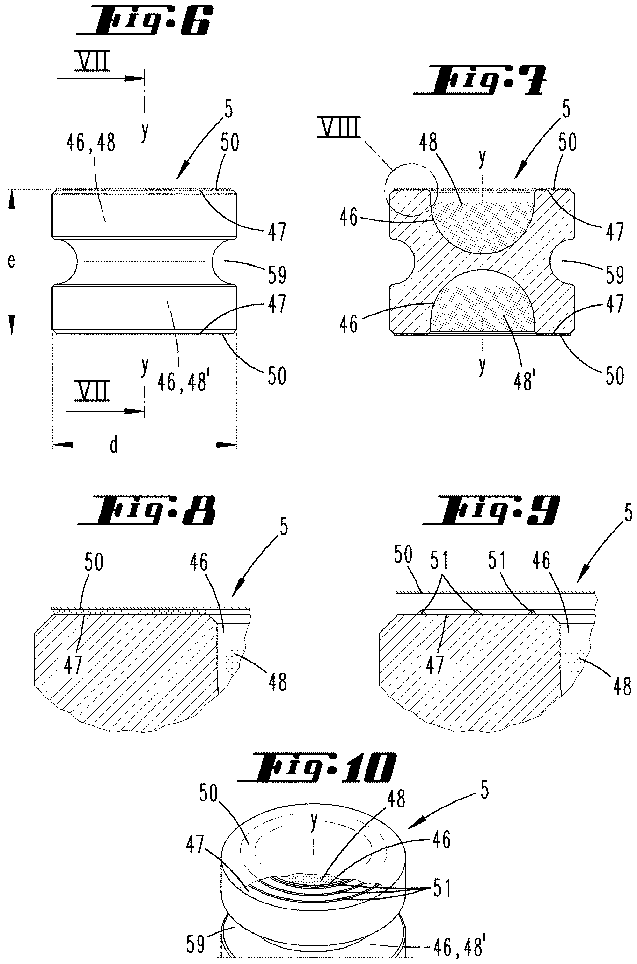

Der Außendurchmesser d kann, wie dargestellt, größer gewählt sein als die in Achsrichtung betrachtete Höhenerstreckung des Substanzbehälters 5. So kann der Durchmesser d etwa dem 1,2- bis 1,5-Fachen der axialen Höhe e entsprechen (vergleiche Figur 6).

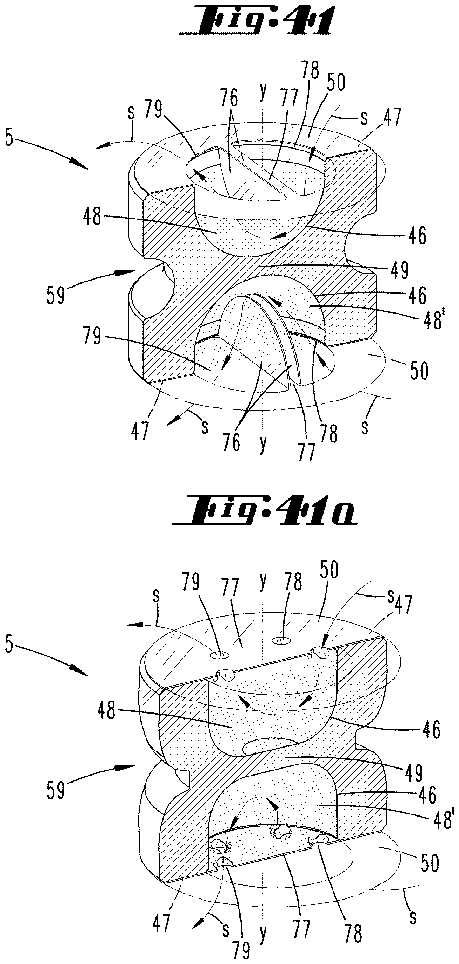

Auch der Substanzbehälter 5 ist bevorzugt aus einem Hartkunststoff gefertigt, beispielsweise Polypropylen oder Polyethylen. Dieser weist zwei in Erstreckungsrichtung der Zylinderachse y gegenüberliegende und konzentrisch zur Zylinderachse y ausgerichtete Teilbereiche 46 beziehungsweise Kavitäten auf. Diese können, wie beispielsweise in Figur 41 dargestellt, im Wesentlichen etwa als halbkugelförmige Vertiefungen gebildet sein. Die jeweilige Öffnung ergibt sich in der quer zur Achse y ausgerichteten jeweiligen Stirnfläche. Jede Kavität kann ausgelegt sein, zur Aufnahme einer Substanzmenge von beispielsweise 2 bis 250 µg, weiter beispielsweise 10 bis 100 µg.

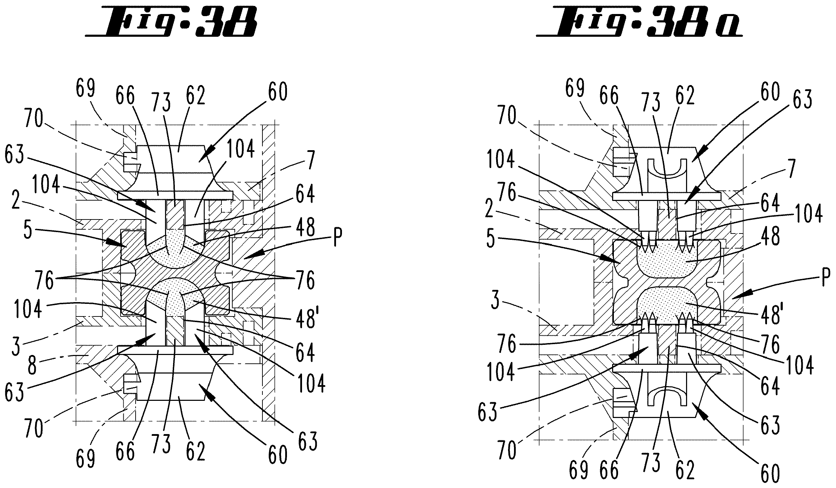

Gemäß den Darstellungen in den Figuren 38a und 41a kann ein Teilbereich 46 des Substanzbehälters 5 auch als im Wesentlichen topfartige Vertiefung ausgebildet sein, mit einer im Wesentlichen relativ zu der Zylinderachse y zylindrischen Topfwandung und einem quer zur Achse y verlaufenden Topfboden. Der Übergang von Topfwandung in den Topfboden ist verrundet. Auch durch diese Querschnittsgeometrie gemäß Figur 41a ist ein günstiges Ausräumen der Kavität erreichbar. Der Topfboden weist einen sich quer zu der Achse y ebenflächig erstreckenden Bereich auf oder ist zumindest mit einem im Vergleich zu dem Übergang von der Topfwandung in den Topfboden sehr viel größeren Radius gekrümmt verlaufend gebildet.

Die Teilbereiche 46 sind ausgebildet zur Aufnahme jeweils einer Substanz 48, 48'.

Durch die Trennung der Teilbereiche 46 zueinander unter Einziehung eines bevorzugt mit Bezug zu der Höhe e mittig eingezogenen Bodens 49, können in den Teilbereichen 46 unterschiedliche Substanzen 48, 48' aufgenommen sein.

Die Kavitäten beziehungsweise Teilbereiche 46 sind jeweils mit einer öffenbaren beziehungsweise durchstechbaren Abdeckung 50 überdeckt. Diese Abdeckungen 50 versiegeln den jeweiligen Teilbereich 46 und die hierin aufgenommene Substanz 48, 48'.

Bei der Abdeckung 50 kann es sich, wie auch bevorzugt, um eine Folie handeln, beispielsweise eine Aluminiumfolie. Diese Folie ist bevorzugt mit dem Stirnrand 47 des Substanzbehälters 5 verschweißt.

Hierzu kann darüber hinaus der Stirnrand 47 umlaufend in Achsrichtung vorstehende Rippen 51 aufweisen, die nach Auflage der folienartigen Abdeckung 50 im Zuge des Schweißvorganges, insbesondere Ultraschweißvorganges, schmelzen und unter Vergleichmäßigung in der Oberfläche die Anhaftung der Abdeckung 50 erbringen.

In der vorrichtungsfesten Führungseinrichtung 4 ist eine Mehrzahl solcher hinsichtlich des Aufbaus und der Abmessungen bevorzugt gleicher Substanzbehälter 5 aufgenommen. Die jeweils aufgenommene Substanz in den Substanzbehältern 5 kann jedoch beispielsweise bezüglich der Zusammensetzung und/oder Menge und/oder Dosierung unterschiedlich sein.

Gemäß dem dargestellten Ausführungsbeispiel können 30 solcher Substanzbehälter 5 in der Endlos-Führungsbahn 38 aufgenommen sein, wobei die Substanzbehälter 5 in Längserstreckung der Führungsbahn 38 betrachtet im Wesentlichen unmittelbar aneinander zur Anlage kommen. Dabei sind die Substanzbehälter 5 jeweils seitlich durch die Seitenwände 37 der Führungseinrichtung 4 geführt.

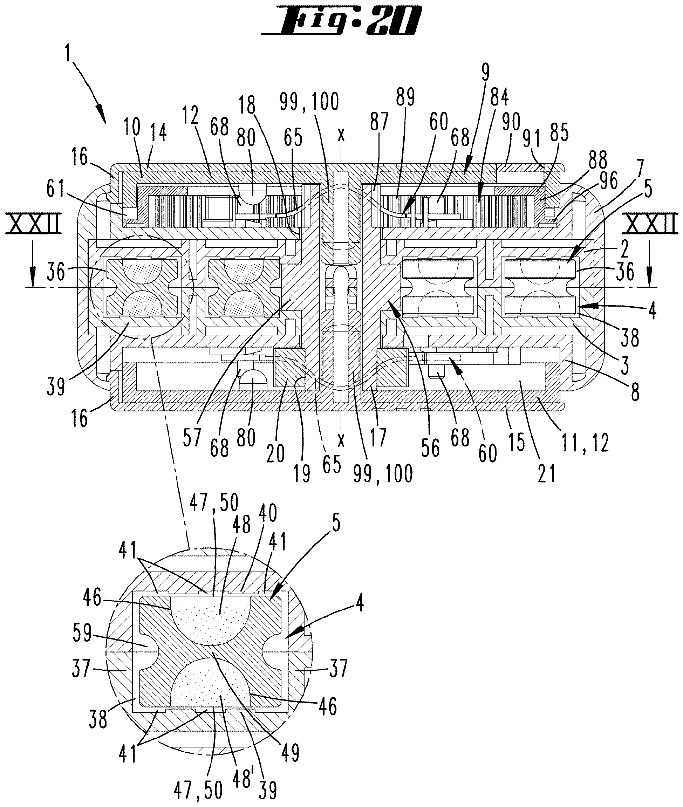

Über ihre Stirnränder 47 erfahren die Substanzbehälter 5 jeweils eine Abstützung an den gegenüber den Längs- und Quernuten 41 und 42 erhabenen Auflageflächen von Bahnboden 39 und Bahndecke 40 (vergleiche Figur 20, insbesondere die dazu gehörige Lupendarstellung).

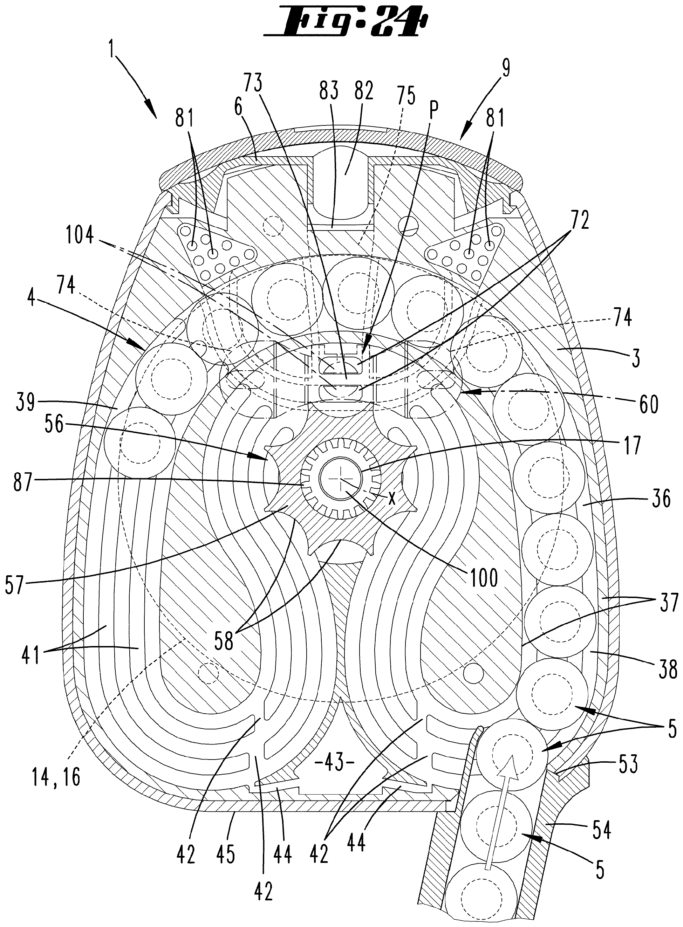

Wie aus der Darstellung in der Figur 24 ersichtlich, können die Substanzbehälter 5 auch erst nach im Wesentlichen vollständigem und funktionsfähigem Zusammenbau der Vorrichtung 1 in die Führungseinrichtung 4 beziehungsweise in die Führungsbahn 38 eingeführt werden. Hierzu ist an dem Gehäuse 52 der Vorrichtung 1, etwa zugeordnet der Stellfläche 45 und somit weiter bevorzugt zugeordnet einem rückwärtigen Wendeabschnitt der Führungsbahn 38, eine Einführ- beziehungsweise Gehäuseöffnung 53 vorgesehen.

An diese Öffnung 53 ist eine Einführhilfe, beispielsweise in Form einer Einführschiene 54, ansetzbar, über welche die in Hintereinanderanordnung in der Schiene aufgenommenen Substanzbehälter 5 allein durch sich unter den Substanzbehältern 5 fortpflanzenden Druck in die Führungsbahn 38 verbracht werden können. Bevorzugt entspricht die Anzahl der in der Einführschiene 54 zunächst aufgenommen Substanzbehältern 5 der in der Führungsbahn 38 beziehungsweise der Führungseinrichtung 4 maximal aufnehmbaren Substanzbehältern 5.

Mit dem letzten eingeführten Substanzbehälter 5 wird eine untereinander nicht verbundene Endloskette aus Substanzbehältern 5 in der Führungseinrichtung 4 geschlossen. Der zuletzt eingeführte Substanzbehälter 5 wirkt in der Endloskette in Art eines Schlusssteines.

Die Einlaufrichtung der Substanzbehälter 5 im Zuge der Bestückung der Vorrichtung 1 unter Nutzung beispielsweise einer solchen Einführschiene 54 entspricht der Verlagerungsrichtung r der Substanzbehälter 5 innerhalb der Vorrichtung 1 bei üblicher Nutzung der Vorrichtung 1.

Die Einführ- beziehungsweise Gehäuseöffnung 53 ist abschließend, das heißt nach vollständiger Bestückung der Vorrichtung 1 mit der vorgegebenen Anzahl an Substanzbehältern 5, durch ein Verschlussteil 55 verschlossen. Das Verschlussteil 55 kann mit dem umlaufenden Gehäuserand beispielsweise verklebt oder verschweißt sein. Gegebenenfalls ist diesbezüglich auch eine Rastverbindung möglich. Wesentlich ist hierbei, dass das Verschlussteil 55 nach einem entsprechenden Verschluss bevorzugt nicht mehr zerstörungsfrei entfernbar ist.

Das Verschlussteil 55 bildet in der Verschlussstellung wandungsinnenseitig einen Teil der Führungsbahn 38 beziehungsweise der Seitenwand 37.

Die Substanzbehälter 5 werden in der Führungseinrichtung 4 beziehungsweise der Führungsbahn 38 über ein Antriebselement 56 in Längserstreckungsrichtung der Führungsbahn 38 bewegt, derart, dass ein entleerter Substanzbehälter 5 aus der Entleerungsposition P heraus verlagert wird und ein unmittelbar nachfolgender, Substanz 48 und 48' in seinen Teilbereichen 46 bevorratender Substanzbehälter 5 in diese Entleerungsposition P nachrückt.

Die Entleerungsposition P ist in dem dargestellten Ausführungsbeispiel erreicht im Zenit der die Schwenkachse x umgreifenden Schleife der Führungsbahn 38.

Das Antriebselement 56 kann, wie auch dargestellt, ein sternradartiges Antriebsrad 57 sein, das drehfest auf der Antriebswelle 17 über das Betätigungsrad 20 antreibbar ist. Bevorzugt ist in der Vorrichtung 1 nur ein solches Antriebselement 56 beziehungsweise Antriebsrad 57 vorgesehen.

Das Antriebsrad 57 ist mit radial offenen Aufnahmeausformungen 58 versehen, die über den Umfang betrachtet durch radial vorstehende Antriebszähne 102 beidseitig begrenzt und durch die Antriebszähne 102 zueinander distanziert sind. Über dem Umfang gleichmäßig verteilt können, wie im Ausführungsbeispiel dargestellt, acht solcher Aufnahmeausformungen 58 vorgesehen sein. Diese sind bevorzugt gleichgestaltet in Form konkaver Randausnehmungen, insbesondere kreisabschnittförmiger Randausnehmungen, deren Radius bevorzugt angepasst ist an den Außendurchmesser d eines Substanzbehälters 5.

Mit den Aufnahmeausformungen 58 erfasst das Antriebselement 56 die Substanzbehälter 5 im Bereich der Führungsbahn-Schleife. Hierbei werden beispielsweise sieben solcher Substanzbehälter 5 erfasst beziehungsweise durch das Antriebsrad 57 geführt und in der Führungsbahn 38 zufolge Drehverlagerung des Antriebsrades 57 geleitet. Durch den sich in der Endloskette unter den Substanzbehältern 5 fortpflanzenden Anlagedruck werden dabei alle Substanzbehälter 5 bei einsprechender Drehung des Antriebsrades 57 weiter bewegt.

Der Drehwinkel des Antriebsrades 57 zum Wechseln der Substanzbehälter 5 in der Entleerungsposition P ist unter anderem abhängig von der Anzahl an Aufnahmeausformungen 58. Bei acht Aufnahmeausformungen 58 ergibt sich ein entsprechender Drehwinkel von bevorzugt etwa 45 Grad.

Wie erwähnt, ist der Schwenkwinkel der Verschlusskappe 9, über welche Schwenkverlagerung über die Antriebswelle 17 auch auf das Antriebsrad 57 Einfluss genommen wird, größer gewählt als der erlaubte Drehwinkel des Antriebsrades 57. Durch die vorbeschriebene Kulissenführung 28 gelangt das Antriebsteil 25 nach Durchführung einer 45 Grad Drehung des Antriebsrades 57 außer Eingriff zu dem Betätigungsrad 20.

Es ist so sichergestellt, dass mit jeder Öffnungsbewegung der Verschlusskappe 9 eine Verlagerung der Substanzbehälter-Kette nur um einen Behälter in Verlagerungsrichtung r vorgenommen wird.

Wie weiter insbesondere aus den Darstellungen in den Figuren 6, 7 und 41 zu erkennen, kann der Substanzbehälter 5 bezogen auf die Zylinderachse y etwa mittig eine außen umlaufende, quer zu der Zylinderachse y ausgerichtete Nut 59 aufweisen. Diese Nut 59 kann mit Bezug auf einen Querschnitt, in welchem sich die Zylinderachse y als Linie darstellt, eine halbkreisförmige sich nach außen öffnende Kontur aufweisen (vergleiche Figur 7 oder 41).

In alternativer Ausgestaltung kann der nach radial innen gerichtete Nutgrund der Nut 59 als ein kreiszylindrischer Wandungsabschnitt gebildet sein, von welchem ausgehend sich die Nutwandungen in einem Querschnitt gemäß Figur 41a nach radial außen jeweils entlang einer gekrümmten Linie erstrecken, derart, dass sich eine trichterartige Erweiterung der Nut 59 bis in die Behälterwandung ergibt.

Die radiale Tiefe der Nut 59 beziehungsweise ein diesbezüglicher Radius der umlaufenden halbkreisförmigen Vertiefung kann bevorzugt so gewählt sein, dass sich insgesamt annähernd eine Vergleichmäßigung der Wandungsstärke des Substanzbehälters 5 ergibt, weiter insbesondere bezüglich der konzentrisch zur Zylinderachse y umlaufenden Wandung.

Durch die gegebene Taillierung des Substanzbehälters 5 ist sogenannten Einfallerscheinigungen, wie diese bei zu hohen Wandungsstärken bei Hartkunststoffprodukten auftreten können, entgegengewirkt. Darüber hinaus ergibt sich hierdurch auch eine Material- und hierüber eine Gewichtsersparnis.

Des Weiteren kann die Nut 59 auch genutzt sein, zur Führung der Substanzbehälter 5 in der Vorrichtung 1, insbesondere in der Führungseinrichtung 4, wozu eine oder beide Seitenwände 37 der Führungsbahn 38 mittig ihrer in Erstreckungsrichtung der Schwenkachse x betrachteten Höhe eine in Richtung auf die gegenüberliegende Seitenwand weisende Rippe oder dergleichen aufweisen kann, die in die Nut 59 des Substanzbehälters 5 führend eingreift. So kann darüber die Führung der Substanzbehälter 5 in der Führungseinrichtung 4 gegebenenfalls allein über diese in die Nuten 59 eingreifenden Rippen erfolgen. Die Stirnränder 47 der Substanzbehälter 5 können dabei zu dem Bahnboden 39 und/oder zu der Bahndecke 40 beabstandet sein.

Zugeordnet der Entleerungsposition P eines Substanzbehälters 5 ist eine Einstecheinrichtung 60 vorgesehen, zur kontrolliert gezielten Öffnung der substanzbehälterseitigen Abdeckung 50.

Entsprechend der Ausbildung von zwei, jeweils Substanzen 48, 48' aufweisenden Teilbereichen 46 in dem Substanzbehälter 5 sind bevorzugt auch zwei Einstecheinrichtungen 60 vorgesehen. Diese liegen in Erstreckungsrichtung der Schwenkachse x in Gegenüberlage.

Dabei kann eine Einrichtung 60 in der Vertiefung 21 des Gehäuse-Schalenunterteiles 8 angeordnet sein und die weitere Einstecheinrichtung 60 in einer solchen Vertiefung 61 im Gehäuse-Schalenoberteil 7.

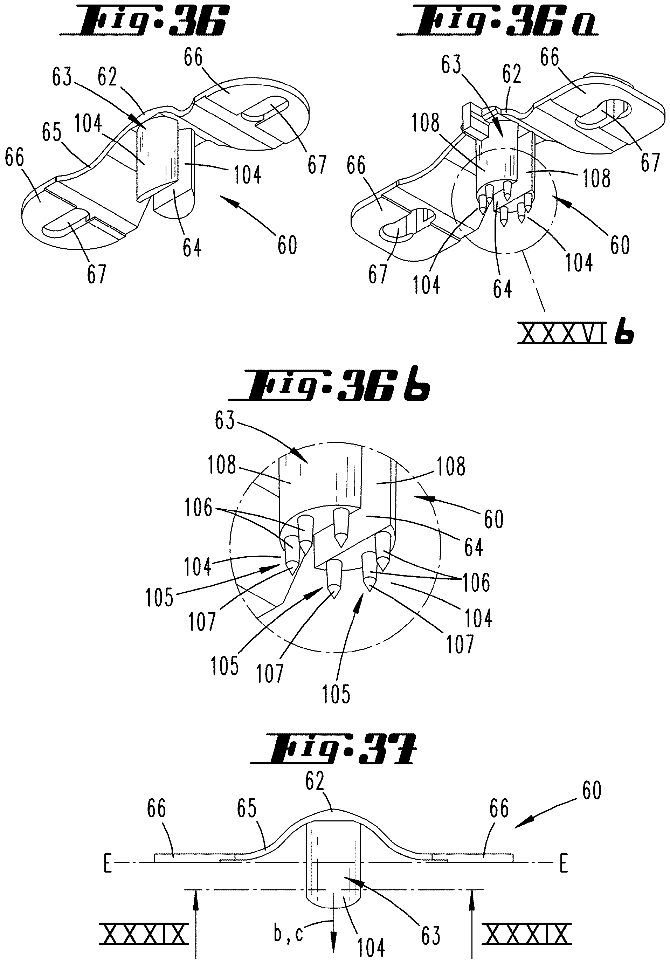

Eine Einstecheinrichtung 60 ist beispielhaft in den Figuren 36 bis 39 dargestellt.

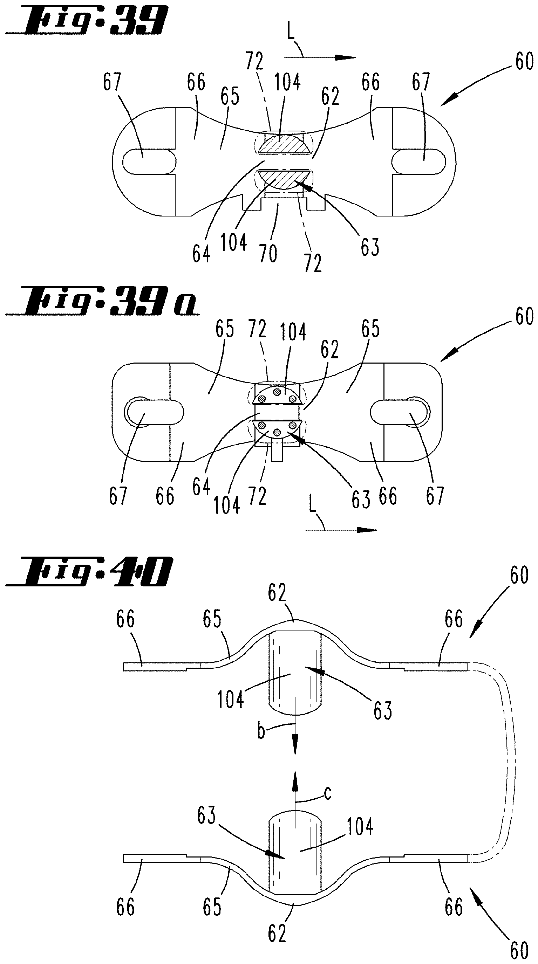

Jede Einstecheinrichtung 60 weist zunächst ein Halteteil 62 auf, an welchem ein Einstechmittel 63 befestigt ist. Bevorzugt sind zugeordnet einem Einstechmittel 63 zwei gesonderte Einstechbereiche 104 vorgesehen, die mit Bezug auf einen Querschnitt gemäß der Darstellung in Figur 39 beispielsweise kreissegmentartig gestaltet sein können.

Wie beispielsweise aus den Figuren 36a und 36b ersichtlich kann jeder Einstechbereich 104 alternativ zwei oder mehr - hier beispielsweise drei - Einstechspitzen 105 aufweisen. Diese können stirnseitig an kreissegmentartigen Sockeln 108 angeordnet sein. Die Einstechspitzen 104 ragen bevorzugt frei über eine Stirnfläche des Sockels 108 hinaus.

Mit Bezug auf eine Längserstreckung L einer Einstecheinrichtung 60 insgesamt sind die Einstechbereiche 104 quer zu dieser Längserstreckung L zueinander beabstandet, wobei bei einer kreissegmentartigen Ausbildung der Einstechbereiche 104 die Flachseiten der Kreissegmente aufeinander zuweisen. Es ergibt sich so entsprechend zwischen den Einstechbereichen 104 ein schlitzartiger Freiraum 64, der sich bevorzugt über die gesamte Erstreckungslänge der Einstechbereiche 104 senkrecht zur Längserstreckung L bis zum Halteteil 62 erstreckt.

Die von dem Halteteil 62 abweisenden freien Endbereiche der Einstechbereiche 104 können klingenartig gespitzt ausgebildet sein, mit einer Klingenspitze bevorzugt jeweils im Zenitbereich der Kreissegmente.

Bei Ausbildung von Einstechspitzen 105 ist eine dornartige Ausbildung bevorzugt, mit einem bevorzugt zylindrischen Bereich 106, über welchen die Einstechspitze 105 an dem Sockel 108 angebunden ist, und einem anschließenden Spitzenbereich 107. Der Spitzenbereich 107 kann ausgehend von dem zylindrischen Bereich 106 konisch sich zum freien Ende hin verjüngend ausgebildet sein.

Jede Einstecheinrichtung 60 weist bevorzugt ein gesondertes Halteteil 62 auf. Wie beispielhaft in Figur 40 anhand der stichpunktierten Linie dargestellt, können aber auch beide Einstecheinrichtungen 60 ein gemeinsames Halteteil 62 aufweisen.

Das Halteteil 62 einer Einstecheinrichtung 60 kann darüber hinaus, wie auch bevorzugt, kombiniert mit einer Kunststofffeder 65 ausgebildet sein. Diese Kunststofffeder 65 weist zwei in Längserstreckung L entgegen gerichtete Federarme 66 auf. Dabei erstrecken sich die Federarme 66 mit Bezug auf die Längserstreckung L in dem jeweiligen Endbereich bevorzugt annähernd innerhalb einer gemeinsamen, quer zur Erstreckungsrichtung der Einstechmittel 63 ausgerichteten Auflageebene E. Der in Längserstreckung L betrachtete mittige Verbindungsbereich der Federarme 66 erstreckt sich in einer Seitenansicht gemäß Figur 37, in der sich eine Einstechrichtung b beziehungsweise c linienartig darstellt und beide Federarme 66 sich in ihrer Längserstreckung abbilden, konkav gewölbt, wobei das Halteteil 62 bevorzugt mittig der Längserstreckung L zwischen den Federarmen 66 angeordnet ist. Die Einstechmittel 63 sind bevorzugt unterseitig der durch die konkave Gestaltung im Bereich des Halteteils 62 gegebenen Gewölbedecke angeordnet und durchsetzen bevorzugt die vorbeschriebene gemeinsame Auflageebene E der beiden Federarme 66 (vergleiche Figur 37).

Die Federarme 66 können ausgehend von ihren freien Enden, jeweils in den, die Auflageebene E anbietenden Bereichen, langlochartige Führungsausnehmungen 67 aufweisen, die jeweils mit einem gehäusefesten Zapfen 68 zusammenwirken können. Über diese Zapfen 68 kann eine Festlegung der Einstecheinrichtung 60 an dem jeweiligen Gehäuseteil (Gehäuse-Schalenoberteil 7 oder Gehäuse-Schalenunterteil 8) erreicht sein. Darüber hinaus kann hierüber auch zugleich eine Führung des Halteteils 62 bei einem Einstechvorgang gegeben sein.

Eine solche Führung kann auch gegeben sein durch Zusammenwirkung zwischen einem gehäuseseitigen Führungsfortsatz 69 und einer im Bereich des Halteteils 62 vorgesehenen randseitigen Führungsausklinkung 70.

Jedenfalls ist jeweils in Einstechrichtung b beziehungsweise c der jeweiligen Einstecheinrichtung 60 eine exakte Führung insbesondere der Einstechmittel 63 gegeben, so dass die aufeinander zugerichteten Einstechrichtung b, c über den gesamten Verlagerungsweg bevorzugt senkrecht gerichtet ist zur Auflageebene E.

Jede Einstecheinrichtung 60 wird bei entsprechender Beaufschlagung gegen die Rückstellkraft der Kunststofffeder 65 in Richtung der Schwenkachse x durch die jeweilige Abdeckung 50 des Substanzbehälters 5 zum Öffnen der Teilbereiche 46 gedrückt. Hierzu sind im jeweiligen Vertiefungsboden des die Einstecheinrichtung 60 tragenden Gehäuse-Schalenoberteils 7 und des Gehäuse-Schalenunterteils 8 Führungsdurchbrüche 71 vorgesehen, durch welche die Einstechbereiche 104 tauchen können.

Auch in dem Gehäuse-Innenoberteil 2 und dem Gehäuse-Innenunterteil 3 sind entsprechende Durchbrüche 72 ausgebildet. Diese sind zunächst angepasst an den Außendurchmesser der Einstechmittel 63 bohrungsartig vorgesehen, hierbei mittig entlang einer Durchmesserlinie einen die Bohrung in zwei Teilabschnitten trennenden Steg 73 aufweisend. Der Steg 73 ist hinsichtlich seiner quer zum Durchmessermaß betrachteten Breite angepasst an das entsprechende Abstandsmaß der Einstechbereiche 104 im Bereich der schlitzartigen Führung 64 zueinander. Ein solcher Steg kann auch im Bereich der Führungsdurchbrüche 71 in den Gehäuseschalenteilen vorgesehen sein.

Der Steg 73 bietet zunächst insbesondere im Zuge des Einstechvorganges eine Stabilisierung und Führung der Einstechbereiche 104. Darüber hinaus bietet der Steg 73 zugleich eine Trennung zwischen Ansaugkanälen 74 und einem Austragskanal 75.

Jeder Kavität beziehungsweise jedem Teilbereich 46 des in der Entleerungsposition P befindlichen Substanzbehälters 5 sind über die jeweiligen Stege 73 getrennt Ansaugkanäle 74 und ein Auftragskanal 75 zugeordnet.

Zufolge der vorbeschriebenen Anordnung und Ausgestaltung der Einstecheinrichtung 60 wirken die Einstechmittel 63 beziehungsweise die Einstechbereiche 104 bevorzugt in entgegengesetzten Einstechrichtungen b und c, jeweils gerichtet entlang der Schwenkachse x. Bevorzugt wirken die Einstechmittel 63 bei entsprechender Belastung zum Durchbruch der Abdeckungen 50 in aufeinander zuweisenden Richtungen.

Durch die Einstechbereiche 104 können sich in jeder Abdeckung 50 des in der Entleerungsposition P befindlichen Substanzbehälters 5 bei einer Ausbildung der Einstechbereiche 104 beispielsweise gemäß der Darstellung in Figur 36 oder 39 zwei kreissegmentartige Stanzabschnitte 76 ergeben, die bevorzugt mittig entlang einer Durchmesserlinie des Substanzbehälters 5 beziehungsweise der Abdeckung 50 über einen verbleibenden Abdeckungssteg 77 nach innen in den jeweiligen Teilbereich 46 einlappen (vergleiche insbesondere Figur 41).

Wie anhand der Figuren 38a und 41a dargestellt, bewirken die Einstechspitzen 105 der Einstecheinrichtung 60 gemäß beispielsweise Figur 36a eine lochartige Stanzung der behälterseitigen Abdeckung 50. Die sich entsprechend ergebenden Stanzlöcher (Öffnungen 78 und 79) können jeweils ein Durchmessermaß aufweisen, welches kleiner gewählt sein kann als beispielsweise 2mm, weiter bevorzugt kleiner als 1,5mm, so gegebenenfalls bis hin zu 0,5mm oder weniger. Es ergeben sich bevorzugt stecknadelgroße Öffnungen 78 und 79.

Zwischen zwei Gruppen an Stanzlöchern, welche Gruppen sich zum einen aus Öffnungen 78 und zum anderen aus Öffnungen 79 zusammensetzen, ergibt sich auch hier ein nicht durchsetzter bzw. nicht gestanzter, mittiger Abdeckungssteg 77.

Es kann sich dabei weiter zugeordnet jedem Teilbereich 46 eine Öffnung 78 zum Eintritt der Luftströmung s aus den Ansaugkanälen 74 und eine Öffnung 79 zum Austritt der mit Substanz 48 beziehungsweise 48' aus dem Teilbereich 46 versetzten Luftströmung s in den Austragskanal 75 ergeben.

Bevorzugt liegt der die Ansaugkanäle 74 zu dem Austragskanal 75 trennende Steg im Durchbruch 72 dichtend auf der Abdeckung 50 beziehungsweise dem sich nach Öffnung der Abdeckung 50 ergebenden Abdeckungssteg 77 auf, so dass eine Zwangsführung der Luftströmung s durch die Öffnung 78 und durch den Teilbereich 46 gegeben ist.

Die Ausformung des Teilbereiches 46 als gegebenenfalls halbkugelartige Vertiefung unterstützt den Räumungseffekt über die Luftströmung s. Es ergeben sich keine strömungsmäßigen Todbereiche. Durch die in Richtung auf den Teilbereichgrund einlappenden Stanzabschnitte 76 wird die Luftströmung s bodennah durch den Teilbereich 46 geführt, was das vollständige Räumen des Teilbereiches 46 unterstützt.

Bevorzugt werden beide Einstecheinrichtungen 60 gleichzeitig belastet und entlastet. Auch kann diesbezüglich eine wahlweise Belastung der einen oder beider Einstecheinrichtungen 60 vorgenommen werden.

Dargestellt in den zeichnerischen Darstellungen ist eine Ausführungsform, bei welcher die Bewegung beider Einstecheinrichtungen 60 synchron erfolgt zufolge Schwenkverlagerung der Verschlusskappe 9.

Hierzu kann unterseitig des Verschlusskappen-Oberteils 10 und des Verschlusskappen-Unterteils 11, jeweils gerichtet in Richtung auf die jeweils zugewandte Vertiefung 21 beziehungsweise 61, ein Nocken 80 angeformt sein, der im Zuge der Schwenkbewegung der Verschlusskappen 9, bevorzugt nachgeordnet einer Vorverlagerung des Substanzbehälters 5 in die Entleerungsposition P, die Einstechmittel 63 gegen die Rückstellkraft der Kunststofffeder 65 in Einstechrichtung b und c niederdrücken. Hierbei beaufschlagen die Nocken 80 die Einstecheinrichtung 60 bevorzugt im Bereich des jeweiligen Halteteils 62.

Der vorbeschriebene Vorlauf des Substanzbehälters 5 zur Erlangung der Entleerungsposition P ist beispielsweise bereits mit einer Schwenkverlagerung der Verschlusskappe 9 um etwa 45 Grad erreicht. Unter Aufhebung der Mitschleppbewegung zwischen Verschlusskappe 9 und Betätigungsrad 20 kann die Verschlusskappe 9 hiernach frei weiter in Richtung auf die vollständige Öffnungsstellung verschwenken, wobei im Zuge dieser Schwenkbewegung die Nocken 80 die Halteteile 62 der Einstecheinrichtungen 60, diese beaufschlagend, überlaufen. Zufolge des Vorlaufes des Substanzbehälters 5 ist hierbei sichergestellt, dass ein neuer, nicht geräumter Substanzbehälter 5 in der vorbeschriebenen Entleerungsposition P vorliegt. Erst hiernach erfolgt eine gezielte Durchstoßung der Abdeckungen 50.

Zum Ende der Verschwenkbewegung der Verschlusskappe 9 verlassen die Nocken 80 den Einflussbereich auf die Einstecheinrichtungen 60, welche sich zufolge der Rückstellkraft der Kunststofffedern 65 wieder zurück in ihrer Ausgangsstellung bewegen. Hierbei fahren die Einstechmittel 63 beziehungsweise Einstechbereiche 104 wieder aus den Teilbereichen 46 des Substanzbehälters 5 heraus, zur entsprechenden Freigabe der Öffnungen 78 und 79 beziehungsweise zur strömungsmäßigen Verbindung dieser Öffnungen mit den Ansaug- und Austragskanälen 74, 75.

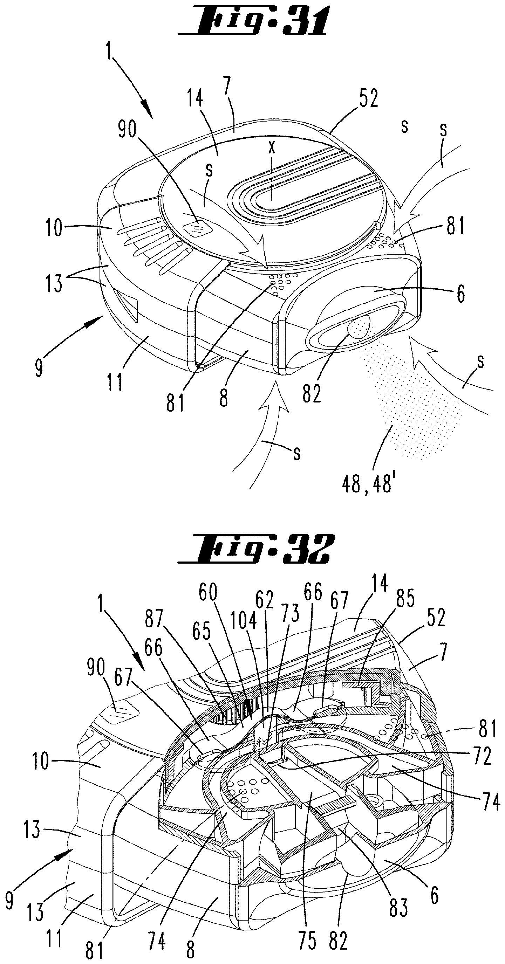

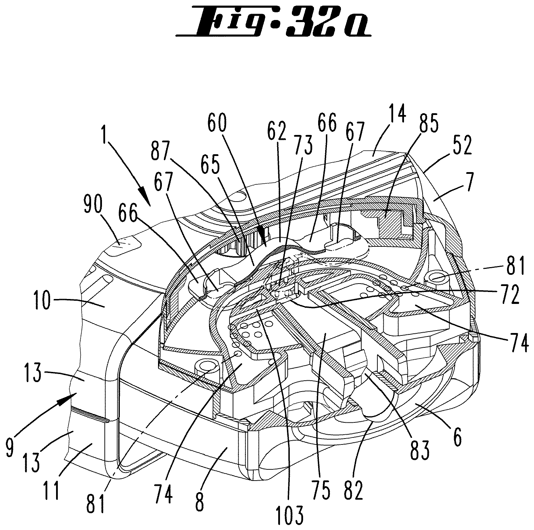

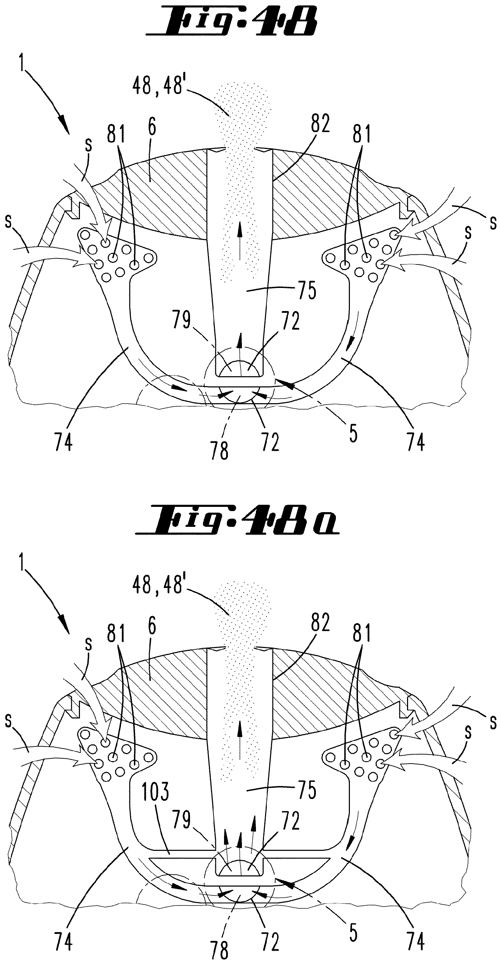

Jeden Teilbereich 46 des in der Entleerungsposition befindlichen Substanzbehälters 5 sind zwei Ansaugkanäle 74 zugeordnet. Deren Ansaugöffnungen 81 sind beidseitig des Mundstückes 6 in dem jeweiligen Gehäuse-Schalenoberteil 7 beziehungsweise dem Gehäuse-Schalenunterteil 8 ausgebildet, während die Ansaugkanäle 74 im Wesentlichen sich in dem Gehäuse-Innenoberteil 2 beziehungsweise dem Gehäuse-Innenunterteil 3 ausformend erstrecken können (vergleiche beispielsweise Figuren 32 und 48).

Es ergeben sich so insgesamt in der Vorrichtung 1 vier Ansaugkanäle 74 mit vier Ansaugöffnungen 81, welche Ansaugöffnungen 81 so beidseitig neben dem Mundstück 6 positioniert sind, dass diese erst nach einem Aufschwenken der Verschlusskappe 9 in Richtung auf die Offenstellung freigelegt werden. In der verschlossenen Grundstellung der Vorrichtung 1 liegen die Ansaugöffnungen 81 geschützt unter der Verschlusskappe 9 versteckt.

Die beiden Ansaugkanäle 74 einer Kavität treffen sich bevorzugt unmittelbar im Bereich des durch den Steg 73 getrennten Durchbruches 72 (vergleiche auch Figur 48).

In alternativer Ausgestaltung kann, insbesondere und bevorzugt im Zusammenhang mit der Nutzung einer Einstecheinrichtung 60 mit Einstechspitzen 105 gemäß den Figuren 36a, 36b und 39a, ein die Ansaugkanäle 74 in Luftströmungsrichtung s vor dem Substanzbehälter 5 bzw. vor der Entleerungsposition P verbindender Bypass 103 vorgesehen sein (siehe Fig. 32a und 48a). Durch diesen Querkanal (Bypass 103) kann ein Teil der angesaugten Luft im Zuge eines Inhalationsvorganges unmittelbar und ohne Durchsetzung der Kavitäten des Substanzbehälters 5 in den Austragskanal 75 strömen. Ein solcher Bypass 103 kann, wie auch bevorzugt, jedem Paar der Ansaugkanäle 74 zugeordnet sein.

Weiter ist jeder Kavität beziehungsweise jedem Teilbereich 46 eines in der Entleerungsstellung befindlichen Substanzbehälters 5 ein Austragskanal 75 zugeordnet. Entsprechend ergeben sich in der Vorrichtung 1 insgesamt zwei Austragskanäle 75, die zunächst ausgehend von der zugeordneten Durchbruchöffnung getrennt zueinander in Richtung auf das Mundstück 6 geführt sind, dies insbesondere in eine Richtung etwa senkrecht zu der vorbeschriebenen rückwärtigen Stellfläche 45.

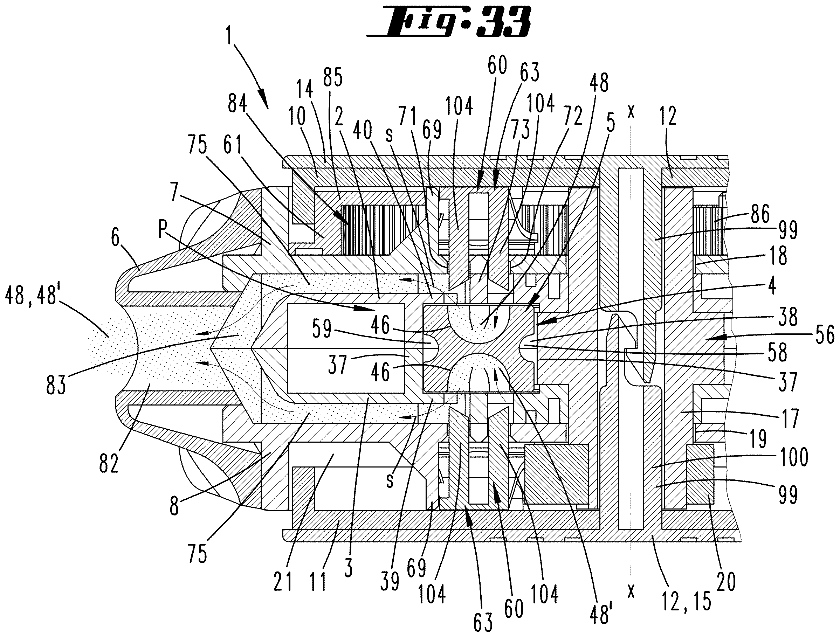

Die beiden Austragskanäle 75 sind unmittelbar im Übergang zu dem Mundstückkanal 82 zusammengeführt (vergleiche beispielsweise Figur 33). In diesem Zusammenführungsbereich 83 kann gegebenenfalls ein Verwirbelungselement oder dergleichen vorgesehen sein.

Entsprechend der vorbeschriebenen Ausgestaltung und Trennung der Austragskanäle 75 werden im Zuge eines Inhalationsvorganges nach Öffnen der Abdeckungen 50 durch die Einstecheinrichtungen 60 und Aufbau einer Luftströmung s zufolge Ansaugen beziehungsweise Einatmen über das Mundstück 6 die Substanzen 48 und 48' getrennt aus den jeweiligen Teilbereichen 46 geräumt und erst unmittelbar vor Übergang in den Atembereich des Benutzers, insbesondere im Zusammenführungsbereich 83 im Wurzelbereich des Mundstückes 6, zusammengeführt und vermengt beziehungsweise verwirbelt.

Die Vorrichtung 1 ist weiter ausgelegt und ausgebildet zur Zählung der durchgeführten oder noch verbleibenden Entleerungsvorgänge beziehungsweise Inhalationsvorgänge. Hierzu ist ein Zählwerk 84 vorgesehen.

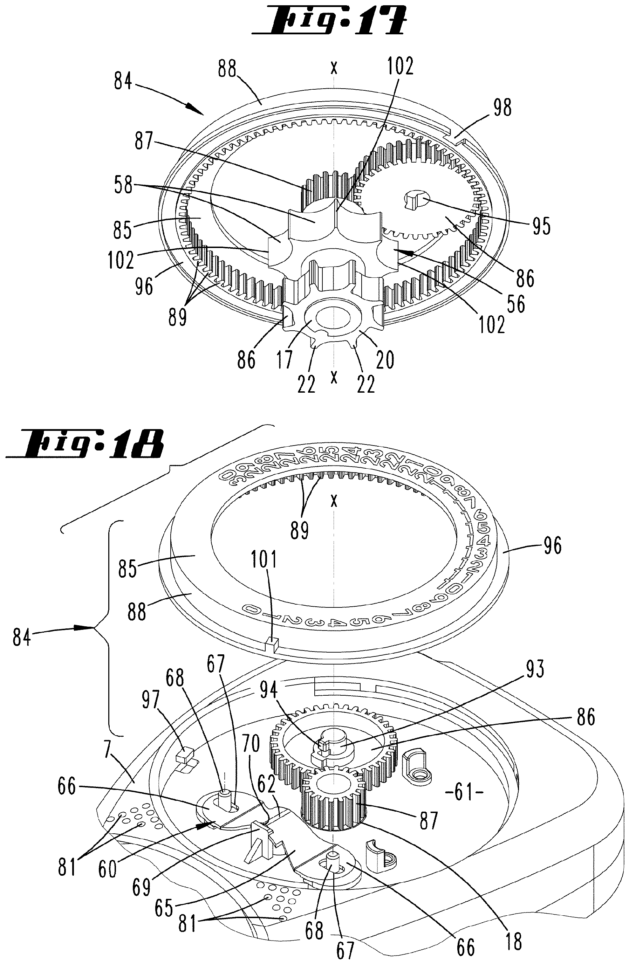

Das Zählwerk 84 weist im Wesentlichen, wie beispielsweise aus den Detaildarstellungen in den Figuren 17 und 18 zu erkennen, ein ringförmiges Zählrad 85, ein Übertragungszahnrad 86 und ein Antriebsritzel 87 auf. Das Antriebsritzel 87 ist drehfest auf der Antriebswelle 17 angeordnet und kämmt mit einer Außenverzahnung des Übertragungszahnrades 86. Die Außenverzahnung des Übertragungszahnrades 86 wiederum kämmt mit einer innenseitig eines umlaufenden Kragens 88 des Zählrades 85 ausgebildeten Innenverzahnung 89.

Das so gebildete, getriebeartige Zählwerk 84 ist im Wesentlichen angeordnet zwischen dem Gehäuse-Schalenoberteil 7 und dem zugeordneten Verschlusskappen-Oberteil 10.

Im Bereich einer oberseitigen, ringförmigen Fläche des Zählrades 85 sind Symbole, insbesondere Ziffern aufgebracht. Bevorzugt entspricht die Anzahl der Ziffern der Anzahl der in der Führungseinrichtung 4 der Vorrichtung 1 aufnehmbaren Substanzbehälter 5. So kann, gemäß dem dargestellten Ausführungsbeispiel, eine Ziffernfolge von 0 bis 30 vorgesehen sein.

Die aktuelle Drehausrichtung des Zählrades 85 und entsprechend die darzustellende aktuelle Zahl an beispielsweise noch vorhandenen unverbrauchten Substanzbehältern 5 oder alternativ an bereits verbrauchten Substanzbehältern 5 ist über ein transparentes Fenster 90 im Deckelteil 16 von außen für den Benutzer sichtbar. Das Fenster 90 verschließt einen angepassten Durchbruch 91 im Deckelteil 16. Auch das Verschlusskappen-Oberteil 10 weist einen solchen Durchbruch 92 auf, der in der Mundstück-Verschlussstellung der Verschlusskappe 9 in Bezug zu der Schwenkachse x in fluchtender Übereinanderlage zu dem Durchbruch 91 und dem Fenster 90 im Deckelteil 14 sich befindet. Zudem kann ein weiterer, in Umfangsrichtung versetzt ausgebildeter Durchbruch 92' vorgesehen sein, durch welchen die Anzeige auch in der Verschlusskappenoffenstellung erkennbar ist.

Im Zuge der Verschlusskappen-Schwenkbewegung sind die Ziffern des Zählrades 85 nicht über das Fenster 90 sichtbar, da sich der ansonsten geschlossene Deckabschnitt 12 des Verschlusskappen-Oberteils 10 zwischen Zählrad 85 und Fenster 90 stellt. Mit vollendeter Rückschwenkbewegung der Verschlusskappe 9 in die Verschlussstellung des Mundstückes 6 ist die gegenüber vor Einleitung einer Verschwenkung der Verschlusskappe 9 aus der Verschlussstellung in die Öffnungsstellung sichtbare Zahl um 1 erhöht oder alternativ um 1 verringert.

Die Verschlusskappen-Schwenkbewegung bewirkt demzufolge sowohl eine Verlagerung der Substanzbehälter 5 innerhalb der Führungseinrichtung 4 um eine Position, um so den nächsten Substanzbehälter 5 in die Entleerungsposition P zu verbringen, als auch das Öffnen der Abdeckungen 50 beider Teilbereiche 46 des in der Entleerungsposition P befindlichen Substanzbehälters 5 und darüber hinaus eine Änderung der Anzeige des Zählwerke 84.

Das Übertragungszahnrad 86 des Zählwerks 84 ist geführt auf einem Achszapfen 93 des Gehäuse-Schalenoberteils 7. Dabei erstreckt sich eine geometrische Drehachse des Übertragungszahnrades 86 gleichgerichtet zur Schwenkachse x.

Der Achszapfen 93 weist endseitig, entsprechend beabstandet zu dem Vertiefungsboden, in welchem der Achszapfen 93 wurzelt, einen Radialvorsprung 94 auf. Das Übertragungszahnrad 86 weist eine entsprechend angepasste, schlüssellochartige zentrale Durchbrechung auf, die es erlaubt, dass Übertragungszahnrad 86 nur in einer Drehausrichtung auf den Achszapfen 93 aufzuschieben. In der Betriebsstellung unterläuft die Nabe des Übertragungszahnrades 86 den Radialvorsprung 94 des Achszapfens 93, so dass das Übertragungszahnrad 86 frei drehbar ist.

Das Zählrad 85 weist entlang seines umlaufenden Kragens 88 einen nach außen weisenden Radialkragen 86 auf. Dieser Radialkragen 86 ist in der Betriebsstellung überfangen von einem im Bereich einer die Vertiefung 61 umfassenden Gehäusewandung nach radial innen abragenden, eine weitere Ausrichtungsausformung 97 ausbildenden Steg. Im Bereich des Radialkragens 96 des Zählrades 85 ist eine an die Ausrichtungsausformung 97 angepasste randoffene Ausnehmung 98 vorgesehen.

Zufolge der vorbeschriebenen Ausgestaltungen sind sowohl das Zählrad 85 als auch das Übertragungszahnrad 86 nur in einer vorgegebenen Winkelausrichtung relativ zueinander und/oder relativ zu dem Antriebsritzel 87 montierbar.

Dabei kann die Einbauposition insbesondere des Zählrades 85 derart sein, dass erst nach Einführen der Substanzbehälter 5 in die ansonsten betriebsbereite Vorrichtung 1 das Zählrad 85 so ausgerichtet wird, dass durch das Fenster 90 beispielsweise die dann noch maximale Anzahl durchführbarer Inhalation beziehungsweise unbenutzter Substanzbehälter 5 erkennbar ist, so gemäß dem gezeigten Ausführungsbeispiel die Ziffer 30. Durch die in die Führungseinrichtung 4 beispielsweise über die Einführschiene 54 sukzessive eingeführten Substanzbehälter 5 wird das in einer vorgegebenen Drehposition eingesetzte Zählrad 85 über die vorbeschriebene Getriebeanordnung mitgeschleppt und in die exakte Ausgangsposition versetzt.

Nach einem Öffnen der Kavitäten des in der Endlosreihe letzten Substanzbehälters 5 - und bevorzugt einer hiernach durchgeführten Inhalation - kann die Vorrichtung 1 zur Vorbereitung einer vermeintlich nächsten Inhalation gesperrt sein.

So kann in dieser Position das Zählwerk 84, wie auch bevorzugt, Null anzeigen. Das entsprechend in diese Position gedrehte Zählrad 85 kann dabei mit einer im Bereich des Radialkragens 96 angeformten Anschlagrippe 101 gegen einen gehäusefesten Abschnitt, beispielsweise gegen die Ausrichtungsausformung 97, sperrend treten.

Hierdurch kann eine Blockierung des getriebeartigen Zählwerks 84 und hierüber der Antriebswelle 17 erreicht sein, so dass bei einem Versuch eines Aufschwenkens der Verschlusskappe 9 aus der Verschlussstellung heraus in Richtung auf die Offenstellung das Antriebsteil 25 gegen die blockierte Antriebswelle 17 beziehungsweise das drehfest damit verbundene Betätigungsrad 20 tritt.

Die Vorrichtung 1 ist nach Entleerung aller Substanzbehälter 5 gesperrt und bevorzugt nicht weiter nutzbar. Durch die bevorzugte Endlos-Aneinanderreihung der Substanzbehälter 5 in der Führungseinrichtung 4 würde ohne eine solche Blockierung nach dem letzten Substanzbehälter 5 der erste, bereits entleerte Substanzbehälter erneut in die Entleerungsposition P verbracht werden. Einer solchen Fehlbedienung ist durch die vorbeschriebene Blockierung entgegengewirkt.

## Patentansprüche

1. Substanzbehälter (5) für eine Vorrichtung (1) zum Inhalieren pulverförmiger Substanzen (48, 48'), **dadurch gekennzeichnet, dass** der Substanzbehälter (5) zwei Teilbereiche (46) aufweist, die jeweils gesondert eine Menge an Substanz (48, 48') aufweisen, und dass beide Teilbereiche (46) eine öffenbare durchstechbare Abdeckung (50) aufweisen.

2. Substanzbehälter nach Anspruch 1, **dadurch gekennzeichnet, dass** der Substanzbehälter (5) im Wesentlichen zylinderförmig gebildet ist, wobei, bevorzugt, der Substanzbehälter (5) bezogen auf eine Zylinderlängsachse (y) etwa mittig eine außen umlaufende, quer zur Zylinderlängsachse (y) ausgerichtete Nut (59) aufweist, und/oder der Substanzbehälter (5) aus einem Hartkunststoff besteht und/oder die Abdeckung (50) aus einer Folie besteht, beispielsweise einer Aluminiumfolie.

3. Substanzbehälter nach Anspruch 2, **dadurch gekennzeichnet, dass** die Folie mit einem Stirnrand (47) des Substanzbehälters (5) verschweißt ist.

4. Vorrichtung (1) zum Inhalieren pulverförmiger Substanzen (48, 48'), wobei die Vorrichtung eine Mehrzahl von sukzessiv in eine Entleerungsstellung bewegbaren Substanzbehältern (5)nach einem der Ansprüche 1 bis 3 aufweist, **gekennzeichnet durch** zur unmittelbaren Anlage aneinander in einer vorrichtungsfesten Führungseinrichtung (4) aufgenommene untereinander unverbundene Substanzbehälter (5), die durch sich unter den Substanzbehältern (5) fortpflanzenden Anlagedruck bewegbar sind, wobei weiter die Führungseinrichtung (4) ein Antriebsrad (57) mit durch Antriebszähne (102) getrennten Aufnahmeausformungen (58) aufweist und ein Substanzbehälter (5) in der Entleerungsstellung sich in einer Aufnahmeausformung (58) des Antriebsrads (57) befindet.

5. Vorrichtung nach Anspruch 4, **dadurch gekennzeichnet, dass** die Substanzbehälter (5) in der Führungseinrichtung (4) eine endlose Reihe bilden.

6. Vorrichtung nach einem der Ansprüche 4 oder 5, **dadurch gekennzeichnet, dass** ein Antriebselement (56) vorgesehen ist, über das auf einen oder ausgewählte Substanzbehälter (5) zur Bewegung der Substanzbehälter (5) einwirkbar ist, wobei, bevorzugt, das Antriebselement (56) als Antriebsrad (57) ausgebildet ist, mit radial offenen Aufnahmeausformungen (58), zur Einwirkung einer Aufnahmeausformung (58) auf jeweils einen Substanzbehälter (5) und/oder das Antriebselement (56) mit einem von dem Benutzer bewegbaren Antriebsteil (25) in einer Transportrichtung (r) bewegbar ist.

7. Vorrichtung nach Anspruch 6, **dadurch gekennzeichnet, dass** das Antriebselement (56) koaxial und drehverbunden zu einem Betätigungsrad (20) angeordnet ist, wobei, bevorzugt, das Antriebselement (56) und das Betätigungsrad (20) drehfest auf einer Antriebswelle (17) angeordnet sind.

8. Vorrichtung nach Anspruch 7, **dadurch gekennzeichnet, dass** auf das Betätigungsrad (20) mit dem Antriebsteil (25) einwirkbar ist, wobei das Betätigungsrad (20) weiter zur Verhinderung einer Rückdrehung mit einer Rücklaufsperre (23) zusammenwirkt.

9. Vorrichtung nach einem der Ansprüche 7 oder 8, **dadurch gekennzeichnet, dass** das Antriebsteil (25) mit einer Verschlusskappe (9) verbunden ist, so dass bei Bewegung der Verschlusskappe (9) auf einem Verschwenkweg in eine Öffnungsstellung eine Bewegung des Antriebsteils (25) in Transportrichtung (r) erfolgt, wobei, bevorzugt, das Antriebsteil (25) mit Hilfe einer Kulissenführung (28) nur über einen Teilbereich des Verschwenkweges der Verschlusskappe (9) mit dem Betätigungsrad (20) in Eingriff ist.

10. Vorrichtung nach einem der Ansprüche 6 bis 9, **dadurch gekennzeichnet, dass** das Antriebsteil (25) ausfederbar ausgebildet ist.

11. Vorrichtung nach einem der Ansprüche 9 oder 10, **dadurch gekennzeichnet, dass** das Antriebsteil (25) in einer Verschlussstellung der Verschlusskappe (9) sich in einer Ausgangsstellung befindet, mit geringer oder fehlender Einfederung.

12. Vorrichtung nach einem der Ansprüche 4 bis 11, **dadurch gekennzeichnet, dass** die Führungseinrichtung (4) eine Führungsbahn (38) aufweist, die mit einer verschließbaren Einführöffnung (53) für Substanzbehälter (5) in dem Gehäuse (52) in Verbindung steht, wobei, bevorzugt, die Einführöffnung (53) durch ein nur zerstörend entfernbares Verschlussteil (55) verschließbar ist, wobei, weiter bevorzugt, die Einführöffnung (53) durch ein nur zerstörend entfernbares Verschlussteil (55) verschließbar ist.

13. Vorrichtung nach Anspruch 12, **dadurch gekennzeichnet, dass** die Führungsbahn (38) zugeordnet einer Einstecheinrichtung (60) für einen Substanzbehälter (5) Längsnuten (41) aufweist, in denen sich eventuell in der Einstecheinrichtung (60) ausgetretene Substanz (48, 48') sammeln kann, wobei, bevorzugt, ein Sammelraum (43) ausgebildet ist, in dem in den Längsnuten (41) transportierte Substanz (48, 48') auffangbar ist.

14. Verfahren zum Befüllen einer Vorrichtung (1) nach einem der Ansprüche 4 bis 13, **dadurch gekennzeichnet, dass** in einem ersten Verfahrensschritt die Substanzbehälter (5) bei bereits im Wesentlichen montierter Vorrichtung (1) durch eine gesonderte Gehäuseöffnung (53) in einen Vorratsraum (36) der Vorrichtung (1) eingebracht werden, dass die Führungseinrichtung (4) in Form einer Führungsbahn (38) ausgebildet ist, dass die Substanzbehälter (5) in die Führungsbahn (38) in einer auch bei üblicher Nutzung der Vorrichtung (1) gegebenen Transportrichtung (r) eingeführt werden und dass in einem zweiten Verfahrensschritt die gesonderte Gehäuseöffnung (53) durch das ohne Zerstörung nicht mehr entfernbare Verschlussteil (55) verschlossen wird.

15. Verfahren nach Anspruch 14, **dadurch gekennzeichnet, dass** die Substanzbehälter (5) untereinander unverbunden sind und sich nur unter den Substanzbehältern (5) fortpflanzenden Anlagedruck bewegt werden.

## Claims

1. A substance container (5) for a device (1) for inhaling powdery substances (48, 48'), **characterised in that** the substance container (5) has two sub-regions (46) which each separately have a quantity of substance (48, 48'), and **in that** both sub-regions (46) have an openable pierceable cover (50).

2. A substance container according to claim 1, **characterised in that** the substance container (5) is substantially cylindrical, wherein, preferably, the substance container (5) has an outer circumferential groove (59) oriented transversely approximately centrally, to a longitudinal axis (y) of the cylinder, and/or the substance container (5) consists of a hard plastic and/or the cover (50) consists of a foil, for example an aluminium foil.

3. A substance container according to claim 2, **characterised in that** the film is welded to an end edge (47) of the substance container (5).

4. Device (1) for inhaling powdery substances (48, 48'), the device having a plurality of substance containers (5) according to one of claims 1 to 3 which can be moved successively into an emptying position, **characterised by** substance containers (5) which are not connected to one another and are accommodated in a guide device (4) fixed to the device in order to bear directly against one another, which are movable by contact pressure propagating under the substance containers (5), the guide device (4) further having a drive wheel (57) with receiving recesses (58) separated by drive teeth (102) and a substance container (5) in the emptying position being located in a receiving recess (58) of the drive wheel (57).

5. Device according to claim 4, **characterised in that** the substance containers (5) form an endless row in the guide device (4).

6. Device according to one of the claims 4 or 5, **characterised in that** a drive element (56) is provided, by means of which one or selected substance containers (5) can be acted upon for moving the substance containers (5), wherein, preferably, the drive element (56) is designed as a drive wheel (57), with radially open receiving formations (58), for acting a receiving formation (58) on a respective substance container (5) and/or the drive element (56) is movable in a transport direction (r) with a drive part (25) movable by the user.

7. Device according to claim 6, **characterised in that** the drive element (56) is arranged coaxially and rotationally connected to an actuating wheel (20), wherein, preferably, the drive element (56) and the actuating wheel (20) are arranged rotationally fixed on a drive shaft (17).

8. A device according to claim 7, **characterised in that** the actuating wheel (20) is engageable with the drive member (25), the actuating wheel (20) further cooperating with a backstop (23) to prevent reverse rotation.

9. Device according to one of the claims 7 or 8, **characterised in that** the drive part (25) is connected to a closure cap (9), so that when the closure cap (9) is moved on a pivoting path into an open position, a movement of the drive part (25) takes place in the transport direction (r), wherein, preferably, the drive part (25) is engaged with the actuating wheel (20) by means of a link guide (28) only over a partial region of the pivoting path of the closure cap (9).

10. Device according to one of the claims 6 to 9, **characterised in that** the drive part (25) is designed to be deflectable.

11. Device according to one of the claims 9 or 10, **characterised in that** the drive part (25) is in an initial position in a closure position of the closure cap (9), with little or no deflection.

12. Apparatus according to any one of claims 4 to 11, **characterised in that** the guide means (4) comprises a guide track (38) which communicates with a closable insertion opening (53) for substance containers (5) in the housing (52), wherein, preferably, the insertion opening (53) is closable by a closure member (55) which is only destructively removable, wherein, further preferably, the insertion opening (53) is closable by a closure member (55) which is only destructively removable.

13. Device according to claim 12, **characterised in that** the guide track (38) has, associated with an insertion device (60) for a substance container (5), longitudinal grooves (41) in which substance (48, 48') which may have escaped in the insertion device (60) can collect, wherein, preferably, a collecting chamber (43) is formed in which substance (48, 48') transported in the longitudinal grooves (41) can be collected.

14. Method for filling a device (1) according to one of claims 4 to 13, **characterised in that** in a first method step the substance containers (5) are introduced through a separate housing opening (53) into a storage space (36) of the device (1) when the device (1) is already substantially assembled, **in that** the guide device (4) is designed in the form of a guide track (38), **in that** the substance containers (5) are introduced into the guide track (38) in a transport direction (r) which is also given during normal use of the device (1), and **in that**, in a second method step, the separate housing opening (53) is closed by the closure part (55) which can no longer be removed without destruction.

15. Method according to claim 14, **characterised in that** the substance containers (5) are not connected to one another and are only moved under the contact pressure propagating through the substance containers (5).

## Revendications

1. Récipient de substance (5) pour un dispositif (1) d'inhalation de substances pulvérulentes (48, 48'), **caractérisé en ce que** le récipient de substance (5) présente deux zones partielles (46), qui présentent chacune séparément une quantité de substance (48, 48'), et **en ce que** les deux zones partielles (46) présentent un couvercle (50) perforable pouvant être ouvert.

2. Récipient de substance selon la revendication 1, **caractérisé en ce que** le récipient de substance (5) est formé essentiellement en forme de cylindre, le récipient de substance (5) présentant, de préférence, par rapport à un axe longitudinal (y) du cylindre, à peu près au milieu, une rainure (59) périphérique extérieure, orientée transversalement à l'axe longitudinal (y) du cylindre, et/ou le récipient de substance (5) étant constitué d'une matière plastique dure et/ou le couvercle (50) étant constitué d'une feuille, par exemple une feuille d'aluminium.

3. Récipient de substance selon la revendication 2, **caractérisé en ce que** la feuille est soudée à un bord frontal (47) du récipient de substance (5).

4. Dispositif (1) pour l'inhalation de substances pulvérulentes (48, 48'), le dispositif présentant une pluralité de récipients de substance (5) selon l'une des revendications 1 à 3, pouvant être déplacés successivement dans une position de vidage, **caractérisé par** des récipients de substance (5) non reliés entre eux, logés dans un dispositif de guidage (4) solidaire du dispositif, en vue d'un appui direct les uns contre les autres, qui peuvent être déplacés par une pression d'appui se propageant sous les récipients de substance (5), le dispositif de guidage (4) présentant en outre une roue d'entraînement (57) avec des formations de réception (58) séparées par des dents d'entraînement (102) et un récipient de substance (5) se trouvant dans la position de vidage dans une formation de réception (58) de la roue d'entraînement (57).

5. Dispositif selon la revendication 4, **caractérisé en ce que** les récipients de substance (5) forment une rangée sans fin dans le dispositif de guidage (4).

6. Dispositif selon l'une des revendications 4 ou 5, **caractérisé en ce qu'**il est prévu un élément d'entraînement (56) par l'intermédiaire duquel on peut agir sur un récipient de substance (5) ou des récipients de substance (5) sélectionnés pour déplacer les récipients de substance (5), l'élément d'entraînement (56) étant, de préférence, conçu comme une roue d'entraînement (57), avec des formations de réception (58) ouvertes radialement, pour l'action d'une formation de réception (58) sur respectivement un récipient de substance (5) et/ou l'élément d'entraînement (56) peut être déplacé dans une direction de transport (r) avec une partie d'entraînement (25) pouvant être déplacée par l'utilisateur.

7. Dispositif selon la revendication 6, **caractérisé en ce que** l'élément d'entraînement (56) est disposé coaxialement et en liaison de rotation avec une roue d'actionnement (20), dans lequel, de préférence, l'élément d'entraînement (56) et la roue d'actionnement (20) sont disposés de manière solidaire en rotation sur un arbre d'entra nement (17).

8. Dispositif selon la revendication 7, **caractérisé en ce que** l'on peut agir sur la roue d'actionnement (20) avec la partie d'entraînement (25), la roue d'actionnement (20) coopérant en outre avec un dispositif anti-retour (23) pour empêcher une rotation inverse.

9. Dispositif selon l'une des revendications 7 ou 8, **caractérisé en ce que** la partie d'entraînement (25) est reliée à un capuchon de fermeture (9), de sorte que lors du déplacement du capuchon de fermeture (9) sur un chemin de pivotement dans une position d'ouverture, il se produit un déplacement de la partie d'entraînement (25) dans la direction de transport (r), la partie d'entraînement (25) étant, de préférence, en prise avec la roue d'actionnement (20) à l'aide d'un guidage à coulisse (28) uniquement sur une zone partielle du chemin de pivotement du capuchon de fermeture (9).

10. Dispositif selon l'une quelconque des revendications 6 à 9, **caractérisé en ce que** la partie d'entraînement (25) est conçue de manière à pouvoir être suspendue.

11. Dispositif selon l'une des revendications 9 ou 10, **caractérisé en ce que**, dans une position de fermeture du capuchon (9), la pièce d'entraînement (25) est dans une position initiale, avec peu ou pas de compression.

12. Dispositif selon l'une des revendications 4 à 11, **caractérisé en ce que** le dispositif de guidage (4) comporte une voie de guidage (38) qui communique avec une ouverture d'introduction (53) pouvant être fermée pour des récipients de substance (5) dans le boîtier (52), dans lequel, de préférence, l'ouverture d'introduction (53) peut être fermée par une pièce de fermeture (55) ne pouvant être enlevée que par destruction, dans lequel, de manière encore plus préférée, l'ouverture d'introduction (53) peut être fermée par une pièce de fermeture (55) ne pouvant être enlevée que par destruction.

13. Dispositif selon la revendication 12, **caractérisé en ce que** la voie de guidage (38) présente, en association avec un dispositif d'insertion (60) pour un récipient de substance (5), des rainures longitudinales (41) dans lesquelles peut s'accumuler la substance (48, 48') éventuellement sortie dans le dispositif d'insertion (60), un espace collecteur (43) étant de préférence formé, dans lequel peut être recueillie la substance (48, 48') transportée dans les rainures longitudinales (41).

14. Procédé de remplissage d'un dispositif (1) selon l'une des revendications 4 à 13, **caractérisé en ce que**, dans une première étape du procédé, les récipients de substance (5) sont introduits dans un espace de stockage (36) du dispositif (1) par une ouverture séparée du boîtier (53) lorsque le dispositif (1) est déjà essentiellement monté, **en ce que** le dispositif de guidage (4) est réalisé sous la forme d'une voie de guidage (38), **en ce que** les récipients de substance (5) sont introduits dans la voie de guidage (38) dans une direction de transport (r) donnée même en cas d'utilisation habituelle du dispositif (1) et **en ce que**, dans une deuxième étape de procédé, l'ouverture de boîtier (53) séparée est fermée par la pièce de fermeture (55) qui ne peut plus être enlevée sans destruction.

15. Procédé selon la revendication 14, **caractérisé en ce que** les récipients de substance (5) ne sont pas reliés entre eux et ne sont déplacés que sous la pression de contact se propageant dans les récipients de substance (5).
